# EUROPÄISCHE PATENTSCHRIFT

(11) **EP 2 739 725 B1**
(45) Veröffentlichungstag und Bekanntmachung des Hinweises auf die Patenterteilung: **06.11.2019**
(21) Anmeldenummer: 12753905.4
(22) Anmeldetag: 01.08.2012
(51) Int. Cl.: C12N 7/02, C12N 7/04, C07K 14/025, C07K 14/005

(54) **VERFAHREN ZUR AUFREINIGUNG VON VIRUS-ÄHNLICHEN PARTIKELN (VLP)**
METHOD FOR PURIFYING VIRUS-LIKE PARTICLES
PROCÉDÉ DE NETTOYAGE DE PARTICULES SEMBLABLE À UN VIRUS (VLP)

(30) Priorität: 02.08.2011 EP 11176295
(43) Veröffentlichungstag der Anmeldung: 11.06.2014
(73) Patentinhaber: Life Science Inkubator Betriebs GmbH & Co. KG, 53175 Bonn (DE)
(72) Erfinder: DEMINA, Victoria, 53175 Bonn (DE); MANNINGA, Heiko, 53175 Bonn (DE)
(74) Vertreter: von Renesse, Dorothea
(86) Internationale Anmeldenummer: PCT/EP2012/003273
(87) Internationale Veröffentlichungsnummer: WO 2013/017272

(56) Entgegenhaltungen:
- EP-A1- 2 133 358
- EP-A2- 1 270 586
- WO-A1-97/19174
- CITKOWICZ A ET AL: "Characterization of virus-like particle assembly for DNA delivery using asymmetrical flow field-flow fractionation and light scattering", ANALYTICAL BIOCHEMISTRY, ACADEMIC PRESS INC, NEW YORK, Bd. 376, Nr. 2, 15. Mai 2008 (2008-05-15), Seiten 163-172, XP022607155, ISSN: 0003-2697, DOI: 10.1016/J.AB.2008.02.011 [gefunden am 2008-02-19]
- LEB V M ET AL: "Modulation of allergen-specific T-lymphocyte function by virus-like particles decorated with HLA class II molecules", JOURNAL OF ALLERGY AND CLINICAL IMMUNOLOGY, MOSBY, INC, US, Bd. 124, Nr. 1, 1. Juli 2009 (2009-07-01), Seiten 121-128, XP026217957, ISSN: 0091-6749, DOI: 10.1016/J.JACI.2009.04.008 [gefunden am 2009-06-04]
- WANG ET AL: "Interaction with 7SL RNA but Not with HIV-1 Genomic RNA or P Bodies Is Required for APOBEC3F Virion Packaging", JOURNAL OF MOLECULAR BIOLOGY, ACADEMIC PRESS, UNITED KINGDOM, Bd. 375, Nr. 4, 13. November 2007 (2007-11-13), Seiten 1098-1112, XP022405101, ISSN: 0022-2836, DOI: 10.1016/J.JMB.2007.11.017
- KIM ET AL: "Immunogenicity of recombinant human immunodeficiency virus type 1-like particles expressing gp41 derivatives in a pre-fusion state", VACCINE, ELSEVIER LTD, GB, Bd. 25, Nr. 27, 9. Juni 2007 (2007-06-09), Seiten 5102-5114, XP022110671, ISSN: 0264-410X
- PALOMARES L A ET AL: "Challenges for the production of virus-like particles in insect cells: The case of rotavirus-like particles", BIOCHEMICAL ENGINEERING JOURNAL, ELSEVIER, AMSTERDAM, NL, Bd. 45, Nr. 3, 15. August 2009 (2009-08-15), Seiten 158-167, XP026144097, ISSN: 1369-703X, DOI: 10.1016/J.BEJ.2009.02.006 [gefunden am 2009-02-20]
- PEIXOTO ET AL: "Downstream processing of triple layered rotavirus like particles", JOURNAL OF BIOTECHNOLOGY, ELSEVIER SCIENCE PUBLISHERS, AMSTERDAM, NL, Bd. 127, Nr. 3, 1. Dezember 2006 (2006-12-01), Seiten 452-461, XP005787038, ISSN: 0168-1656, DOI: 10.1016/J.JBIOTEC.2006.08.002
- VOLPERS CHRISTOPH ET AL: "Assembly of the major and the minor capsid protein of human papillomavirus type 33 into virus-like particles and tubular structures in insect cells", VIROLOGY, Bd. 200, Nr. 2, 1994, Seiten 504-512, XP008148872, ISSN: 0042-6822
- SANTI ET AL: "An efficient plant viral expression system generating orally immunogenic Norwalk virus-like particles", VACCINE, ELSEVIER LTD, GB, Bd. 26, Nr. 15, 15. Februar 2008 (2008-02-15), Seiten 1846-1854, XP022534494, ISSN: 0264-410X, DOI: 10.1016/J.VACCINE.2008.01.053
- Spectrum Labs.com: "Laboratory Dialysis Frequently Asked Questions", , 28. Juni 2010 (2010-06-28), XP002688367, Gefunden im Internet: URL:http://www.spectrumlabs.com/dialysis/F AQ.html [gefunden am 2012-11-30]
- Monika Deckwer ET AL: "RÖMPP Lexikon Biotechnologie und Gentechnik" In: "2. Auflage, 1999 (E-Book EPUB)", 1 January 1999 (1999-01-01), Georg Thieme.Verlag - Thieme webshop, XP55269389, ISBN: 978-3-13-736402-3 pages 181-181,
- SAXENA A ET AL: "Membrane-based techniques for the separation and purification of proteins: An overview", ADVANCES IN COLLOID AND INTERFACE SCIENCE, ELSEVIER, NL, vol. 145, no. 1-2, 30 January 2009 (2009-01-30), pages 1-22, XP025716991, ISSN: 0001-8686, DOI: 10.1016/J.CIS.2008.07.004 [retrieved on 2008-08-05]

### SEQUENCE LISTING

<110> Life Science Inkubator Betriebs GmbH & Co. KG
<120> verfahren zur Aufreinigung von virusähnlichen Partikeln (VLP)
<130> 53730 K
<160> 15
<170> PatentIn version 3.5
<210> 1
   <211> 1065
   <212> DNA
   <213> JC polyomavirus
<220>
   <221> misc_feature
   <222> (1)..(1065)
   <223> capsid protein VP1
<400> 1
   atggccccaa caaaaagaaa aggagaaagg aaggaccccg tgcaagttcc aaaacttctt       60
   ataagaggag gagtagaagt tctagaagtt aaaactgggg ttgactcaat tacagaggta      120
   gaatgctttt taactccaga aatgggtgac ccagatgagc atcttagggg ttttagtaag      180
   tcaatatcta tatcagatac atttgaaagt gactccccaa atagggacat gcttccttgt      240
   tacagtgtgg ccagaattcc actacccaat ctaaatgagg atctaacctg tggaaatata      300
   ctcatgtggg aggctgtgac cttaaaaact gaggttatag gggtgacaag tttgatgaat      360
   gtgcactcta atgggcaagc aactcatgac aatggtgcag ggaagccagt gcagggcacc      420
   agctttcatt ttttttctgt tgggggggag gctttagaat tacagggggt gctttttaat      480
   tacagaacaa agtacccaga tggaacaatt tttccaaaga atgccacagt gcaatctcaa      540
   gtcatgaaca cagagcacaa ggcgtaccta gataagaaca aagcatatcc tgttgaatgt      600
   tgggttcctg atcccaccag aaatgaaaac acaagatatt ttgggacact aacaggagga      660
   gaaaatgttc ctccagttct tcatataaca aacactgcca caacagtgtt gcttgatgaa      720
   tttggtgttg ggccactttg caaaggtgac aacttatact tgtcagctgt tgatgtctgt      780
   ggcatgttta caaacaggtc tggttcccag cagtggagag gactctccag atattttaag      840
   gtgcagctaa ggaaaaggag ggttaaaaac ccctacccaa tttctttcct tcttactgat      900
   ttaattaaca gaaggactcc tagagttgat gggcagccta tgtatggcat ggatgctcaa      960
   gtagaggagg ttagagtttt tgagggaaca gaggagcttc caggggaccc agacatgatg     1020
   agatacgttg acaaatatgg acagttgcag acaaaaatgc tgtaa                     1065
<210> 2
   <211> 1065
   <212> DNA
   <213> synthetic sequence
<220>
   <221> misc_feature
   <222> (1)..(1065)
   <223> VP1 capsid protein; codon optimized
<400> 2
   atggctccca ccaagcgcaa gggcgagccc aaggaccccg tgcaagtgcc caagctgctg 60
   atccgtggtg gtgtcgaggt gctggaagtc aagaccggcg tggactccat taccgaggtg 120
   gagtgcttcc tcacccccga gatgggtgac cctgacgagc acctgagggg cttctccaag 180
   tccatctcca tctccgacac cttcgagtcc gactccccca accgtgacat gctgccctgc 240
   tactccgtgg ctcgtatccc cctgcccaac ctgaacgagg acctgacttg cggcaacatc 300
   ctgatgtggg aggctgtgac cctcaagacc gaggtcatcg gcgtgacttc cctgatgaac 360
   gtgcactcca acggccaggc tacccacgac aacggtgctg gcaagcccgt gcagggaacc 420
   tccttccact tcttctccgt gggtggcgag gctctggaac tccagggcgt ggtgttcaac 480
   taccgtacca agtaccccga cggcaccatc ttccccaaga acgctactgt gcagtcccaa 540
   gtgatgaaca ccgagcacaa ggcttacctg gacaagaaca aggcctaccc cgtggagtgc 600
   tgggtgcccg accccacccg taacgagaac acccgttact tcggcaccct gaccggtgga 660
   gagaacgtgc cccccgtgct gcacatcacc aacaccgcta ccaccgtgct gctggacgag 720
   ttcggtgtcg gtcccctgtg caagggcgac aacctgtacc tgtccgctgt ggacgtgtgc 780
   ggcatgttca ccaaccgttc cggttcccag cagtggcgtg gcctgtcccg ctacttcaag 840
   gtgcagctgc gcaagcgtcg tgtgaagaac ccctacccta tctccttcct gctgaccgac 900
   ctgatcaacc gtcgtacccc tcgtgtggac ggccagccca tgtacggcat ggacgctcag 960
   gtggaagagg tccgcgtgtt cgagggcacc gaggaattgc ccggcgaccc cgacatgatg 1020
   cgttacgtgg acaagtacgg ccagctccag accaagatgc tgtaa 1065
<210> 3
   <211> 354
   <212> PRT
   <213> JC polyomavirus
<220>
   <221> MISC_FEATURE
   <222> (1)..(354)
   <223> capsid protein VP1
<400> 3
   Met Ala Pro Thr Lys Arg Lys Gly Glu Pro Lys Asp Pro Val Gln Val
   1 5 10 15
   Pro Lys Leu Leu Ile Arg Gly Gly Val Glu Val Leu Glu Val Lys Thr
   20 25 30
   Gly Val Asp Ser Ile Thr Glu Val Glu Cys Phe Leu Thr Pro Glu Met
   35 40 45
   Gly Asp Pro Asp Glu His Leu Arg Gly Phe Ser Lys Ser Ile Ser Ile
   50 55 60
   Ser Asp Thr Phe Glu Ser Asp Ser Pro Asn Arg Asp Met Leu Pro Cys
   65 70 75 80
   Tyr Ser Val Ala Arg Ile Pro Leu Pro Asn Leu Asn Glu Asp Leu Thr
   85 90 95
   Cys Gly Asn Ile Leu Met Trp Glu Ala Val Thr Leu Lys Thr Glu Val
   100 105 110
   Ile Gly Val Thr Ser Leu Met Asn Val His Ser Asn Gly Gln Ala Thr
   115 120 125
   His Asp Asn Gly Ala Gly Lys Pro Val Gln Gly Thr Ser Phe His Phe
   130 135 140
   Phe Ser Val Gly Gly Glu Ala Leu Glu Leu Gln Gly Val Val Phe Asn
   145 150 155 160
   Tyr Arg Thr Lys Tyr Pro Asp Gly Thr Ile Phe Pro Lys Asn Ala Thr
   165 170 175
   Val Gln Ser Gln Val Met Asn Thr Glu His Lys Ala Tyr Leu Asp Lys
   180 185 190
   Asn Lys Ala Tyr Pro Val Glu Cys Trp Val Pro Asp Pro Thr Arg Asn
   195 200 205
   Glu Asn Thr Arg Tyr Phe Gly Thr Leu Thr Gly Gly Glu Asn Val Pro
   210 215 220
   Pro Val Leu His Ile Thr Asn Thr Ala Thr Thr Val Leu Leu Asp Glu
   225 230 235 240
   Phe Gly Val Gly Pro Leu Cys Lys Gly Asp Asn Leu Tyr Leu Ser Ala
   245 250 255
   Val Asp Val Cys Gly Met Phe Thr Asn Arg Ser Gly Ser Gln Gln Trp
   260 265 270
   Arg Gly Leu Ser Arg Tyr Phe Lys Val Gln Leu Arg Lys Arg Arg Val
   275 280 285
   Lys Asn Pro Tyr Pro Ile Ser Phe Leu Leu Thr Asp Leu Ile Asn Arg
   290 295 300
   Arg Thr Pro Arg Val Asp Gly Gln Pro Met Tyr Gly Met Asp Ala Gln
   305 310 315 320
   Val Glu Glu Val Arg Val Phe Glu Gly Thr Glu Glu Leu Pro Gly Asp
   325 330 335
   Pro Asp Met Met Arg Tyr Val Asp Lys Tyr Gly Gln Leu Gln Thr Lys
   340 345 350
   Met Leu
<210> 4
   <211> 1035
   <212> DNA
   <213> JC polyomavirus
<220>
   <221> misc_feature
   <222> (1)..(1035)
   <223> cpasid protein VP2
<400> 4
   atgggtgccg cacttgcact tttgggggac ctagttgcta ctgtttctga ggctgctgct 60
   gccacaggat tttcagtagc tgaaattgct gctggagagg ctgctgctac tatagaagtt 120
   gaaattgcat cccttgctac tgtagagggg attacaagta cctctgaggc tatagctgct 180
   ataggcctta ctcctgaaac atatgctgta ataactggag ctccgggggc tgtagctggg 240
   tttgctgcat tggttcaaac tgtaactggt ggtagtgcta ttgctcagtt gggatataga 300
   ttttttgctg actgggatca taaagtttca acagttgggc tttttcagca gccagctatg 360
   gctttacaat tatttaatcc agaagactac tatgatattt tatttcctgg agtgaatgcc 420
   tttgttaaca atattcacta tttagatcct agacattggg gcccgtcctt gttctccaca 480
   atctcccagg ctttttggaa tcttgttaga gatgatttgc cagccttaac ctctcaggaa 540
   attcagagaa gaacccaaaa actatttgtt gaaagtttag caaggttttt ggaagaaact 600
   acttgggcaa tagttaattc accagctaac ttatataatt atatttcaga ctattattct 660
   agattgtctc cagttaggcc ctctatggta aggcaagttg cccaaaggga gggaacctat 720
   atttcttttg gccactcata cacccaaagt atagatgatg cagacagcat tcaagaagtt 780
   acccaaaggc tagatttaaa aaccccaaat gtgcaatctg gtgaatttat agaaagaagt 840
   attgcaccag gaggtgcaaa tcaaagatct gctcctcaat ggatgttgcc tttactttta 900
   gggttgtacg ggactgtaac acctgctctt gaagcatatg aagatggccc caacaaaaag 960
   aaaaggagaa aggaaggacc ccgtgcaagt tccaaaactt cttataagag gaggagtaga 1020
   agttctagaa gttaa 1035
<210> 5
   <211> 1062
   <212> DNA
   <213> synthetic sequence
<220>
   <221> misc_feature
   <222> (1)..(1062)
   <223> capsid protein VP2-HA including haemagglutinin epitop tag, codon optimized <400> 5
<210> 6
   <211> 344
   <212> PRT
   <213> JC polyomavirus
<220>
   <221> MISC_FEATURE
   <222> (1)..(344)
   <223> capsid protein VP2
<400> 6
   Met Gly Ala Ala Leu Ala Leu Leu Gly Asp Leu Val Ala Thr Val Ser
   1               5                   10                  15
   Glu Ala Ala Ala Ala Thr Gly Phe Ser Val Ala Glu Ile Ala Ala Gly
               20                  25                  30
   Glu Ala Ala Ala Thr Ile Glu Val Glu Ile Ala Ser Leu Ala Thr Val
           35                  40                  45
   Glu Gly Ile Thr Ser Thr Ser Glu Ala Ile Ala Ala Ile Gly Leu Thr
   50 55 60
   Pro Glu Thr Tyr Ala Val Ile Thr Gly Ala Pro Gly Ala Val Ala Gly
   65 70 75 80
   Phe Ala Ala Leu Val Gln Thr Val Thr Gly Gly Ser Ala Ile Ala Gln
   85 90 95
   Leu Gly Tyr Arg Phe Phe Ala Asp Trp Asp His Lys Val Ser Thr Val
   100 105 110
   Gly Leu Phe Gln Gln Pro Ala Met Ala Leu Gln Leu Phe Asn Pro Glu
   115 120 125
   Asp Tyr Tyr Asp Ile Leu Phe Pro Gly Val Asn Ala Phe Val Asn Asn
   130 135 140
   Ile His Tyr Leu Asp Pro Arg His Trp Gly Pro Ser Leu Phe Ser Thr
   145 150 155 160
   Ile Ser Gln Ala Phe Trp Asn Leu Val Arg Asp Asp Leu Pro Ala Leu
   165 170 175
   Thr Ser Gln Glu Ile Gln Arg Arg Thr Gln Lys Leu Phe Val Glu Ser
   180 185 190
   Leu Ala Arg Phe Leu Glu Glu Thr Thr Trp Ala Ile Val Asn Ser Pro
   195 200 205
   Ala Asn Leu Tyr Asn Tyr Ile Ser Asp Tyr Tyr Ser Arg Leu Ser Pro
   210 215 220
   Val Arg Pro Ser Met Val Arg Gln Val Ala Gln Arg Glu Gly Thr Tyr
   225 230 235 240
   Ile Ser Phe Gly His Ser Tyr Thr Gln Ser Ile Asp Asp Ala Asp Ser
   245 250 255
   Ile Gln Glu Val Thr Gln Arg Leu Asp Leu Lys Thr Pro Asn Val Gln
   260 265 270
   Ser Gly Glu Phe Ile Glu Arg Ser Ile Ala Pro Gly Gly Ala Asn Gln
   275 280 285
   Arg Ser Ala Pro Gln Trp Met Leu Pro Leu Leu Leu Gly Leu Tyr Gly
   290 295 300
   Thr Val Thr Pro Ala Leu Glu Ala Tyr Glu Asp Gly Pro Asn Lys Lys
   305 310 315 320
   Lys Arg Arg Lys Glu Gly Pro Arg Ala Ser Ser Lys Thr Ser Tyr Lys
   325 330 335
   Arg Arg Ser Arg Ser Ser Arg Ser
   340
<210> 7
   <211> 234
   <212> PRT
   <213> synthetic sequence
<220>
   <221> MISC_FEATURE
   <222> (1)..(234)
   <223> capsid protein VP2-HA including C-terminal haemagglutinin epitope tag, codon optimized
<400> 7
   Met Gly Ala Ala Leu Ala Leu Leu Gly Asp Leu Val Ala Thr Val Ser
   1 5 10 15
   Glu Ala Ala Ala Ala Thr Gly Phe Ser Val Ala Glu Ile Ala Ala Gly
   20 25 30
   Glu Ala Ala Ala Thr Ile Glu Val Glu Ile Ala Ser Leu Ala Thr Val
   35 40 45
   Glu Gly Ile Thr Ser Thr Ser Glu Ala Ile Ala Ala Ile Gly Leu Thr
   50 55 60
   Pro Glu Thr Tyr Ala Val Ile Thr Gly Ala Pro Gly Ala Val Ala Gly
   65 70 75 80
   Phe Ala Ala Leu Val Gln Thr Val Thr Gly Gly Ser Ala Ile Ala Gln
   85 90 95
   Leu Gly Tyr Arg Phe Phe Ala Asp Trp Asp His Lys Val Ser Thr Val
   100 105 110
   Gly Leu Phe Gln Gln Pro Ala Met Ala Leu Gln Leu Phe Asn Pro Glu
   115 120 125
   Asp Tyr Tyr Asp Ile Leu Phe Pro Gly Val Asn Ala Phe Val Asn Asn
   130 135 140
   Ile His Tyr Leu Asp Pro Arg His Trp Gly Pro Ser Leu Phe Ser Thr
   145 150 155 160
   Ile Ser Gln Ala Phe Trp Asn Leu Val Arg Asp Asp Leu Pro Ala Leu
   165 170 175
   Thr Ser Gln Glu Ile Gln Arg Arg Thr Gln Lys Leu Phe Val Glu Ser
   180 185 190
   Leu Ala Arg Phe Leu Glu Glu Thr Thr Trp Ala Ile Val Asn Ser Pro
   195 200 205
   Ala Asn Leu Tyr Asn Tyr Ile Ser Asp Tyr Tyr Ser Arg Leu Ser Pro
   210 215 220
   Val Arg Pro Ser Met Val Arg Gln Val Ala
   225 230
<210> 8
   <211> 678
   <212> DNA
   <213> JC polyomavirus
<220>
   <221> misc_feature
   <222> (1)..(678)
   <223> capsid protein VP3
<400> 8
   atggctttac aattatttaa tccagaagac tactatgata ttttatttcc tggagtgaat 60
   gcctttgtta acaatattca ctatttagat cctagacatt ggggcccgtc cttgttctcc 120
   acaatctccc aggctttttg gaatcttgtt agagatgatt tgccagcctt aacctctcag 180
   gaaattcaga gaagaaccca aaaactattt gttgaaagtt tagcaaggtt tttggaagaa 240
   actacttggg caatagttaa ttcaccagct aacttatata attatatttc agactattat 300
   tctagattgt ctccagttag gccctctatg gtaaggcaag ttgcccaaag ggagggaacc 360
   tatatttctt ttggccactc atacacccaa agtatagatg atgcagacag cattcaagaa 420
   gttacccaaa ggctagattt aaaaacccca aatgtgcaat ctggtgaatt tatagaaaga 480
   agtattgcac caggaggtgc aaatcaaaga tctgctcctc aatggatgtt gcctttactt 540
   ttagggttgt acgggactgt aacacctgct cttgaagcat atgaagatgg ccccaacaaa 600
   aagaaaagga gaaaggaagg accccgtgca agttccaaaa cttcttataa gaggaggagt 660
   agaagttcta gaagttaa 678
<210> 9
   <211> 705
   <212> DNA
   <213> synthetic sequence
<220>
   <221> misc_feature
   <222> (1)..(705)
   <223> capsid protein VP3-HA including haemagglutinin epitop tag, codon optimized
<400> 9
   atggctctgc agctgttcaa ccccgaggac tactacgaca tcctgttccc cggcgtgaac 60
   gccttcgtga acaacatcca ctacctggac ccccggcact ggggccctag cctgttctct 120
   acaatcagcc aggccttctg gaacctcgtg cgggacgatc tgcctgccct gaccagccag 180
   gaaatccagc ggcggaccca gaaactgttc gtggaaagcc tggcccggtt cctggaagag 240
   acaacctggg ccatcgtgaa cagccccgcc aacctgtaca actacatcag cgactactac 300
   agcagactga gccccgtgcg gcccagcatg gtgcgccagg tggcacagag agagggcacc 360
   tatatcagct tcggccactc ctacacccag agcatcgacg acgccgacag catccaggaa 420
   gtgacccaga gactggacct gaaaaccccc aacgtgcaga gcggcgagtt catcgagaga 480
   tccattgccc ctggcggagc caaccagaga tctgcccctc agtggatgct gcccctgctg 540
   ctgggcctgt acggcacagt gacaccagcc ctggaagcct acgaggacgg ccccaacaag 600
   aagaagcgcc ggaaagaggg ccctagagcc agcagcaaga ccagctacaa gcggcggagc 660
   agaagcagca gatcctaccc atacgatgtt ccagattacg cttga 705
<210> 10
   <211> 225
   <212> PRT
   <213> JC polyoma virus
<220>
   <221> MISC_FEATURE
   <222> (1)..(225)
   <223> capsid protein VP3
<400> 10
   Met Ala Leu Gln Leu Phe Asn Pro Glu Asp Tyr Tyr Asp Ile Leu Phe
   1               5                   10                  15
   Pro Gly Val Asn Ala Phe Val Asn Asn Ile His Tyr Leu Asp Pro Arg
               20                  25                  30
   His Trp Gly Pro Ser Leu Phe Ser Thr Ile Ser Gln Ala Phe Trp Asn
           35                  40                  45
   Leu Val Arg Asp Asp Leu Pro Ala Leu Thr Ser Gln Glu Ile Gln Arg
       50                  55                  60
   Arg Thr Gln Lys Leu Phe Val Glu Ser Leu Ala Arg Phe Leu Glu Glu
   65                  70                  75                  80
   Thr Thr Trp Ala Ile Val Asn Ser Pro Ala Asn Leu Tyr Asn Tyr Ile
                   85                  90                  95
   Ser Asp Tyr Tyr Ser Arg Leu Ser Pro Val Arg Pro Ser Met Val Arg
               100                 105                 110
   Gln Val Ala Gln Arg Glu Gly Thr Tyr Ile Ser Phe Gly His Ser Tyr
           115                 120                 125
   Thr Gln Ser Ile Asp Asp Ala Asp Ser Ile Gln Glu Val Thr Gln Arg
       130                 135                 140
   Leu Asp Leu Lys Thr Pro Asn Val Gln Ser Gly Glu Phe Ile Glu Arg
   145                 150                 155                 160
   Ser Ile Ala Pro Gly Gly Ala Asn Gln Arg Ser Ala Pro Gln Trp Met
                   165                 170                 175
   Leu Pro Leu Leu Leu Gly Leu Tyr Gly Thr Val Thr Pro Ala Leu Glu
               180                 185                 190
   Ala Tyr Glu Asp Gly Pro Asn Lys Lys Lys Arg Arg Lys Glu Gly Pro
           195                 200                 205
   Arg Ala Ser Ser Lys Thr Ser Tyr Lys Arg Arg Ser Arg Ser Ser Arg
       210                 215                 220
   Ser
   225
<210> 11
   <211> 234
   <212> PRT
   <213> synthetic sequence
<220>
   <221> MISC_FEATURE
   <222> (1)..(234)
   <223> capsid protein VP3-HA including C-terminal haemagglutinin epitope tag, codon optimized
<400> 11
   Met Ala Leu Gln Leu Phe Asn Pro Glu Asp Tyr Tyr Asp Ile Leu Phe
   1               5                   10                  15
   Pro Gly Val Asn Ala Phe Val Asn Asn Ile His Tyr Leu Asp Pro Arg
               20                  25                  30
   His Trp Gly Pro Ser Leu Phe Ser Thr Ile Ser Gln Ala Phe Trp Asn
           35                  40                  45
   Leu Val Arg Asp Asp Leu Pro Ala Leu Thr Ser Gln Glu Ile Gln Arg
       50                  55                  60
   Arg Thr Gln Lys Leu Phe Val Glu Ser Leu Ala Arg Phe Leu Glu Glu
   65                  70                  75                  80
   Thr Thr Trp Ala Ile Val Asn Ser Pro Ala Asn Leu Tyr Asn Tyr Ile
                   85                  90                  95
   Ser Asp Tyr Tyr Ser Arg Leu Ser Pro Val Arg Pro Ser Met Val Arg
               100                 105                 110
   Gln Val Ala Gln Arg Glu Gly Thr Tyr Ile Ser Phe Gly His Ser Tyr
           115                 120                 125
   Thr Gln Ser Ile Asp Asp Ala Asp Ser Ile Gln Glu Val Thr Gln Arg
       130                 135                 140
   Leu Asp Leu Lys Thr Pro Asn Val Gln Ser Gly Glu Phe Ile Glu Arg
   145                 150                 155                 160
   Ser Ile Ala Pro Gly Gly Ala Asn Gln Arg Ser Ala Pro Gln Trp Met
                   165                 170                 175
   Leu Pro Leu Leu Leu Gly Leu Tyr Gly Thr Val Thr Pro Ala Leu Glu
               180                 185                 190
   Ala Tyr Glu Asp Gly Pro Asn Lys Lys Lys Arg Arg Lys Glu Gly Pro
           195                 200                 205
   Arg Ala Ser Ser Lys Thr Ser Tyr Lys Arg Arg Ser Arg Ser Ser Arg
       210                 215                 220
   Ser Tyr Pro Tyr Asp Val Pro Asp Tyr Ala
   225                 230
<210> 12
   <211> 1596
   <212> DNA
   <213> Human papillomavirus type 16
<220>
   <221> misc_feature
   <222> (1)..(1596)
   <223> capsid protein L1
<400> 12
   atgcaggtga cttttattta catcctagtt attacatgtt acgaaaacga cgtaaacgtt      60
   taccatattt tttttcagat gtctctttgg ctgcctagtg aggccactgt ctacttgcct     120
   cctgtcccag tatctaaggt tgtaagcacg gatgaatatg ttgcacgcac aaacatatat     180
   tatcatgcag gaacatccag actacttgca gttggacatc cctattttcc tattaaaaaa     240
   cctaacaata acaaaatatt agttcctaaa gtatcaggat tacaatacag ggtatttaga     300
   atacatttac ctgaccccaa taagtttggt tttcctgaca cctcatttta taatccagat     360
   acacagcggc tggtttgggc ctgtgtaggt gttgaggtag gtcgtggtca gccattaggt     420
   gtgggcatta gtggccatcc tttattaaat aaattggatg acacagaaaa tgctagtgct     480
   tatgcagcaa atgcaggtgt ggataataga gaatgtatat ctatggatta caaacaaaca     540
   caattgtgtt taattggttg caaaccacct ataggggaac actggggcaa aggatcccca     600
   tgtaccaatg ttgcagtaaa tccaggtgat tgtccaccat tagagttaat aaacacagtt     660
   attcaggatg gtgatatggt tgatactggc tttggtgcta tggactttac tacattacag     720
   gctaacaaaa gtgaagttcc actggatatt tgtacatcta tttgcaaata tccagattat     780
   attaaaatgg tgtcagaacc atatggcgac agcttatttt tttatttacg aagggaacaa     840
   atgtttgtta gacatttatt taatagggct ggtgctgttg gtgaaaatgt accagacgat     900
   ttatacatta aaggctctgg gtctactgca aatttagcca gttcaaatta ttttcctaca     960
   cctagtggtt ctatggttac ctctgatgcc caaatattca ataaacctta ttggttacaa    1020
   cgagcacagg gccacaataa tggcatttgt tggggtaacc aactatttgt tactgttgtt    1080
   gatactacac gcagtacaaa tatgtcatta tgtgctgcca tatctacttc agaaactaca    1140
   tataaaaata ctaactttaa ggagtaccta cgacatgggg aggaatatga tttacagttt    1200
   atttttcaac tgtgcaaaat aaccttaact gcagacgtta tgacatacat acattctatg    1260
   aattccacta ttttggagga ctggaatttt ggtctacaac ctcccccagg aggcacacta    1320
   gaagatactt ataggtttgt aacatcccag gcaattgctt gtcaaaaaca tacacctcca    1380
   gcacctaaag aagatcccct taaaaaatac actttttggg aagtaaattt aaaggaaaag    1440
   ttttctgcag acctagatca gtttccttta ggacgcaaat ttttactaca agcaggattg    1500
   aaggccaaac caaaatttac attaggaaaa cgaaaagcta cacccaccac ctcatctacc    1560
   tctacaactg ctaaacgcaa aaaacgtaag ctgtaa                               1596
<210> 13
   <211> 531
   <212> PRT
   <213> Human papillomavirus type 16
<220>
   <221> MISC_FEATURE
   <222> (1)..(531)
   <223> capsid protein L1
<400> 13
   Met Gln Val Thr Phe Ile Tyr Ile Leu Val Ile Thr Cys Tyr Glu Asn
   1               5                   10                  15
   Asp Val Asn Val Tyr His Ile Phe Phe Gln Met Ser Leu Trp Leu Pro
               20                  25                  30
   Ser Glu Ala Thr Val Tyr Leu Pro Pro Val Pro Val Ser Lys Val Val
           35                  40                  45
   Ser Thr Asp Glu Tyr Val Ala Arg Thr Asn Ile Tyr Tyr His Ala Gly
       50                  55                  60
   Thr Ser Arg Leu Leu Ala Val Gly His Pro Tyr Phe Pro Ile Lys Lys
   65                  70                  75                  80
   Pro Asn Asn Asn Lys Ile Leu Val Pro Lys Val Ser Gly Leu Gln Tyr
                   85                  90                  95
   Arg Val Phe Arg Ile His Leu Pro Asp Pro Asn Lys Phe Gly Phe Pro
   100 105 110
   Asp Thr Ser Phe Tyr Asn Pro Asp Thr Gln Arg Leu Val Trp Ala Cys
   115 120 125
   Val Gly Val Glu Val Gly Arg Gly Gln Pro Leu Gly Val Gly Ile Ser
   130 135 140
   Gly His Pro Leu Leu Asn Lys Leu Asp Asp Thr Glu Asn Ala Ser Ala
   145 150 155 160
   Tyr Ala Ala Asn Ala Gly Val Asp Asn Arg Glu Cys Ile Ser Met Asp
   165 170 175
   Tyr Lys Gln Thr Gln Leu Cys Leu Ile Gly Cys Lys Pro Pro Ile Gly
   180 185 190
   Glu His Trp Gly Lys Gly Ser Pro Cys Thr Asn Val Ala Val Asn Pro
   195 200 205
   Gly Asp Cys Pro Pro Leu Glu Leu Ile Asn Thr Val Ile Gln Asp Gly
   210 215 220
   Asp Met Val Asp Thr Gly Phe Gly Ala Met Asp Phe Thr Thr Leu Gln
   225 230 235 240
   Ala Asn Lys Ser Glu Val Pro Leu Asp Ile Cys Thr Ser Ile Cys Lys
   245 250 255
   Tyr Pro Asp Tyr Ile Lys Met Val Ser Glu Pro Tyr Gly Asp Ser Leu
   260 265 270
   Phe Phe Tyr Leu Arg Arg Glu Gln Met Phe Val Arg His Leu Phe Asn
   275 280 285
   Arg Ala Gly Ala Val Gly Glu Asn Val Pro Asp Asp Leu Tyr Ile Lys
   290 295 300
   Gly Ser Gly Ser Thr Ala Asn Leu Ala Ser Ser Asn Tyr Phe Pro Thr
   305 310 315 320
   Pro Ser Gly Ser Met Val Thr Ser Asp Ala Gln Ile Phe Asn Lys Pro
   325 330 335
   Tyr Trp Leu Gln Arg Ala Gln Gly His Asn Asn Gly Ile Cys Trp Gly
   340 345 350
   Asn Gln Leu Phe Val Thr Val Val Asp Thr Thr Arg Ser Thr Asn Met
   355 360 365
   Ser Leu Cys Ala Ala Ile Ser Thr Ser Glu Thr Thr Tyr Lys Asn Thr
   370 375 380
   Asn Phe Lys Glu Tyr Leu Arg His Gly Glu Glu Tyr Asp Leu Gln Phe
   385 390 395 400
   Ile Phe Gln Leu Cys Lys Ile Thr Leu Thr Ala Asp Val Met Thr Tyr
   405 410 415
   Ile His Ser Met Asn Ser Thr Ile Leu Glu Asp Trp Asn Phe Gly Leu
   420 425 430
   Gln Pro Pro Pro Gly Gly Thr Leu Glu Asp Thr Tyr Arg Phe Val Thr
   435 440 445
   Ser Gln Ala Ile Ala Cys Gln Lys His Thr Pro Pro Ala Pro Lys Glu
   450 455 460
   Asp Pro Leu Lys Lys Tyr Thr Phe Trp Glu Val Asn Leu Lys Glu Lys
   465 470 475 480
   Phe Ser Ala Asp Leu Asp Gln Phe Pro Leu Gly Arg Lys Phe Leu Leu
   485 490 495
   Gln Ala Gly Leu Lys Ala Lys Pro Lys Phe Thr Leu Gly Lys Arg Lys
   500 505 510
   Ala Thr Pro Thr Thr Ser Ser Thr Ser Thr Thr Ala Lys Arg Lys Lys
   515 520 525
   Arg Lys Leu
   530
<210> 14
   <211> 1422
   <212> DNA
   <213> Human papillomavirus type 16
<220>
   <221> misc_feature
   <222> (1)..(1422)
   <223> capsid protein L2
<400> 14
   atgcgacaca aacgttctgc aaaacgcaca aaacgtgcat cggctaccca actttataaa 60
   acatgcaaac aggcaggtac atgtccacct gacattatac ctaaggttga aggcaaaact 120
   attgctgatc aaatattaca atatggaagt atgggtgtat tttttggtgg gttaggaatt 180
   ggaacagggt cgggtacagg cggacgcact gggtatattc cattgggaac aaggcctccc 240
   acagctacag atacacttgc tcctgtaaga ccccctttaa cagtagatcc tgtgggccct 300
   tctgatcctt ctatagtttc tttagtggaa gaaactagtt ttattgatgc tggtgcacca 360
   acatctgtac cttccattcc cccagatgta tcaggattta gtattactac ttcaactgat 420
   accacacctg ctatattaga tattaataat actgttacta ctgttactac acataataat 480
   cccactttca ctgacccatc tgtattgcag cctccaacac ctgcagaaac tggagggcat 540
   tttacacttt catcatccac tattagtaca cataattatg aagaaattcc tatggataca 600
   tttattgtta gcacaaaccc taacacagta actagtagca cacccatacc agggtctcgc 660
   ccagtggcac gcctaggatt atatagtcgc acaacacaac aagttaaagt tgtagaccct 720
   gcttttgtaa ccactcccac taaacttatt acatatgata atcctgcata tgaaggtata 780
   gatgtggata atacattata tttttctagt aatgataata gtattaatat agctccagat 840
   cctgactttt tggatatagt tgctttacat aggccagcat taacctctag gcgtactggc 900
   attaggtaca gtagaattgg taataaacaa acactacgta ctcgtagtgg aaaatctata 960
   ggtgctaagg tacattatta ttatgatttt agtactattg atcctgcaga agaaatagaa 1020
   ttacaaacta taacaccttc tacatatact accacttcac atgcagcctc acctacttct 1080
   attaataatg gattatatga tatttatgca gatgacttta ttacagatac ttctacaacc 1140
   ccggtaccat ctgtaccctc tacatcttta tcaggttata ttcctgcaaa tacaacaatt 1200
   ccttttggtg gtgcatacaa tattccttta gtatcaggtc ctgatatacc cattaatata 1260
   actgaccaag ctccttcatt aattcctata gttccagggt ctccacaata tacaattatt 1320
   gctgatgcag gtgactttta tttacatcct agttattaca tgttacgaaa acgacgtaaa 1380
   cgtttaccat attttttttc agatgtctct ttggctgcct ag 1422
<210> 15
   <211> 473
   <212> PRT
   <213> Human papillomavirus type 16
<220>
   <221> MISC_FEATURE
   <222> (1)..(473)
   <223> capsid protein L2
<400> 15
   Met Arg His Lys Arg Ser Ala Lys Arg Thr Lys Arg Ala Ser Ala Thr
   1               5                   10                  15
   Gln Leu Tyr Lys Thr Cys Lys Gln Ala Gly Thr Cys Pro Pro Asp Ile
               20                  25                  30
   Ile Pro Lys Val Glu Gly Lys Thr Ile Ala Asp Gln Ile Leu Gln Tyr
           35                  40                  45
   Gly Ser Met Gly Val Phe Phe Gly Gly Leu Gly Ile Gly Thr Gly Ser
       50                  55                  60
   Gly Thr Gly Gly Arg Thr Gly Tyr Ile Pro Leu Gly Thr Arg Pro Pro
   65                  70                  75                  80
   Thr Ala Thr Asp Thr Leu Ala Pro Val Arg Pro Pro Leu Thr Val Asp
   85 90 95
   Pro Val Gly Pro Ser Asp Pro Ser Ile Val Ser Leu Val Glu Glu Thr
   100 105 110
   Ser Phe Ile Asp Ala Gly Ala Pro Thr Ser Val Pro Ser Ile Pro Pro
   115 120 125
   Asp Val Ser Gly Phe Ser Ile Thr Thr Ser Thr Asp Thr Thr Pro Ala
   130 135 140
   Ile Leu Asp Ile Asn Asn Thr Val Thr Thr Val Thr Thr His Asn Asn
   145 150 155 160
   Pro Thr Phe Thr Asp Pro Ser Val Leu Gln Pro Pro Thr Pro Ala Glu
   165 170 175
   Thr Gly Gly His Phe Thr Leu Ser Ser Ser Thr Ile Ser Thr His Asn
   180 185 190
   Tyr Glu Glu Ile Pro Met Asp Thr Phe Ile Val Ser Thr Asn Pro Asn
   195 200 205
   Thr Val Thr Ser Ser Thr Pro Ile Pro Gly Ser Arg Pro Val Ala Arg
   210 215 220
   Leu Gly Leu Tyr Ser Arg Thr Thr Gln Gln Val Lys Val Val Asp Pro
   225 230 235 240
   Ala Phe Val Thr Thr Pro Thr Lys Leu Ile Thr Tyr Asp Asn Pro Ala
   245 250 255
   Tyr Glu Gly Ile Asp Val Asp Asn Thr Leu Tyr Phe Ser Ser Asn Asp
   260 265 270
   Asn Ser Ile Asn Ile Ala Pro Asp Pro Asp Phe Leu Asp Ile Val Ala
   275 280 285
   Leu His Arg Pro Ala Leu Thr Ser Arg Arg Thr Gly Ile Arg Tyr Ser
   290 295 300
   Arg Ile Gly Asn Lys Gln Thr Leu Arg Thr Arg Ser Gly Lys Ser Ile
   305 310 315 320
   Gly Ala Lys Val His Tyr Tyr Tyr Asp Phe Ser Thr Ile Asp Pro Ala
   325 330 335
   Glu Glu Ile Glu Leu Gln Thr Ile Thr Pro Ser Thr Tyr Thr Thr Thr
   340 345 350
   Ser His Ala Ala Ser Pro Thr Ser Ile Asn Asn Gly Leu Tyr Asp Ile
           355                 360                 365
   Tyr Ala Asp Asp Phe Ile Thr Asp Thr Ser Thr Thr Pro Val Pro Ser
       370                 375                 380
   Val Pro Ser Thr Ser Leu Ser Gly Tyr Ile Pro Ala Asn Thr Thr Ile
   385                 390                 395                 400
   Pro Phe Gly Gly Ala Tyr Asn Ile Pro Leu Val Ser Gly Pro Asp Ile
                   405                 410                 415
   Pro Ile Asn Ile Thr Asp Gln Ala Pro Ser Leu Ile Pro Ile Val Pro
               420                 425                 430
   Gly Ser Pro Gln Tyr Thr Ile Ile Ala Asp Ala Gly Asp Phe Tyr Leu
           435                 440                 445
   His Pro Ser Tyr Tyr Met Leu Arg Lys Arg Arg Lys Arg Leu Pro Tyr
       450                 455                 460
   Phe Phe Ser Asp Val Ser Leu Ala Ala
   465                 470

## Beschreibung

Die vorliegende Erfindung betrifft ein Verfahren zur Aufreinigung von VLP enthaltenden Zusammensetzungen und hochreine VLP enthaltende Zusammensetzungen sowie deren Verwendung in diagnostischen oder therapeutischen Verfahren.

Bei der Entwicklung spezifischer diagnostischer oder therapeutischer Verfahren hat die Verwendung von Transfersystemen (delivery systems), die einen möglichst zellspezifischen Transfer von Substanzen wie Nukleinsäuren, Markern oder Wirkstoffen erlauben, eine große Bedeutung. Für diesen zellspezifischen Transfer wurde unter anderem ein System auf Basis von Virus "ähnlichen Partikeln" (VLP, *virus like particles*) entwickelt (WO 97/19174; EP 1 270 586 B1). Grundlage dieses Systems ist die Eigenschaft der VLP, z.B. Wirkstoffe oder Nukleinsäuren verpacken zu können und diese dann spezifisch in bestimmte Zellen einzuschleusen.

VLP können zum Beispiel durch rekombinante Expression des Hauptstrukturproteins VP1 des humanen Polyomavirus JCV hergestellt werden (VP1-VLP). Im Gegensatz zur VP1-Expression anderer Polyomaviren werden die VP1-VLP in den Überstand der Wirtszellenkulturen sezerniert. Zur Aufreinigung der VP1-VLP aus dem Zellkulturüberstand sind bereits mehrere Verfahren entwickelt worden. Diese sind aber alle nicht geeignet, VLP in kommerziellem Maßstab (large scale) herzustellen. Dies gilt insbesondere dann, wenn der Herstellungsprozess GMP zertifizierbar sein soll, da dann an die Reinheit des VLP besonders hohe Anforderungen gestellt werden.

So beschreiben Goldmann et al. (J. Virol., 1999, 73: 4465-4469) die Aufreinigung von in Insektenzellen exprimierten VP1-VLP durch Dichtezentrifugation mit einer 40%igen Sucroselösung gefolgt von einer Dichtezentrifugation mit 40% Sucrose und 50% Metrizamid (2-({3-(acetylamino)-5-[acetyl(methyl)amino]-2,4,6-triiodobenzoyl}amino)-2-deoxy-D-glucopyranose. Dieses Verfahren ist nicht nur für die large scale Produktion ungeeignet. Auch sind die so bereitgestellten VP1-Proteine mit VP1-Fragmenten von 38 und 40 kDa verunreinigt.

Zur Aufreinigung rekombinanter VLP aus lysierten E. coli-Zellen verwenden Pushko et al. (Protein Engineering, 1993, 6(8): 883-891) eine Ammoniumsulfatpräzipitation mit anschließender Gelpermeationschromatographie unter Verwendung einer Sephadex G25-Säule und einer G100 Säule.

Aus WO 92/13081 A1 ist ein Aufreinigungsverfahren für die Isolierung von MS-2 abgeleiteten VLP durch fraktionierte Ammoniumsulfatfällung und anschließender isoelektrischer Punkt-Präzipitation, Sucrose-Dichtezentrifugation und Gelpermeations-chromatographie bekannt.

In der WO 2006/136566 A9 wird die Aufreinigung rekombinanter bakteriell exprimierter VLP über eine Anionenaustauscherchromatographie gefolgt von einer Hydroxylapatitsäule und einer optionalen Gelpermeationschromatographie beschrieben.

Citkowicz et al. (Anal. Biochem., 2008, 376(2): 163-172) beschreiben ein VLP-Aufreinigungsverfahren, bei dem die VLP zunächst durch Zentrifugation von den Zellrückständen abgetrennt werden und anschließend der VP1-VLP-enthaltende Überstand mit Hilfe von PEG präzipitiert wird. Danach wird mittels eines 0,22 µm Celluloseacetatfilters filtriert. Die VLP werden im Filtrat gesammelt und weiter aufgereinigt.

Die aus diesem Stand der Technik bekannten Verfahren sind für eine Herstellung der VLP außerhalb des Labormaßstabs nur unzureichend geeignet, da sie entweder sehr aufwändig sind, keine Aufskalierung an ein großtechnisches Verfahren erlauben und/oder die hohen Anforderungen eines GMP-konformen Prozesses nicht erfüllen. Letzteres gilt vor allem auch aufgrund von Verunreinigungen, die unter anderem in den VLP eingeschlossen sein können.

Der Erfindung liegt daher die Aufgabe zugrunde, ein Herstellungsverfahren für VLP bereitzustellen, das zumindest einen Nachteil der bekannten Herstellungsverfahren reduziert oder sogar weitgehend vermeidet.

Diese Aufgabe wir erfindungsgemäß durch das Verfahren nach dem Hauptanspruch gelöst. Vorteilhafte Ausführungsformen sind Gegenstand entsprechender Unteransprüche. Weiterhin werden VLP Zusammensetzungen einer bestimmten Reinheit offenbart; ebenso wie vorteilhafte VP1 Proteine.

Die vorliegende Erfindung stellt ein Verfahren zur effektiven Aufreinigung von VLP bereit. Das Verfahren hat den besonderen Vorteil, dass eine large scale Produktion erlaubt wird, die auch den hohen Anforderungen an einen GMP-Prozess ("Gute Herstellungspraxis") genügt.

Das erfindungsgemäße Verfahren zur Aufreinigung von VLP zeichnet sich dadurch aus, dass eine VLP-enthaltende Zusammensetzung über ein Filtermedium, insbesondere eine Membran, filtriert wird, wobei das Filtermedium eine Ausschlussgrenze (Molecular Weight Cut off (MWCO) von mehr als 30 kDa bis 1500 kDa besitzt und als VLP-enthaltende Zusammensetzung der Zellkulturüberstand VLP-exprimierender Zellen verwendet wird. Die Erfinder haben festgestellt, dass mit einem solchen Schritt zu Beginn eines downstream Prozesses - der aus dem Stand der Technik allenfalls nach mindestens einem Chromatographie-Schritt durchgeführt wird - eine einfache und effiziente Aufreinigung der VLP möglich ist.

Bei der Filtration der VLP durch das erfindungsgemäß eingesetzte Filtermedium passieren mögliche Verunreinigungen das Filtermedium und fallen im Filtrat an, während die hochmolekularen VLP in gereinigter Form im Retentat zurückbleiben. Dieses einstufige Verfahren ermöglicht somit eine einfache Abtrennung der VLP von den in der Zusammensetzung vorliegenden Verunreinigungen.

Je nach Verfahrensführung kann in diesem Aufreinigungsschritt eine Konzentrierung der VLP-enthaltenden Zusammensetzung erreicht werden, da während der Filtration naturgemäß das Flüssigkeitsvolumen des Retentats abnimmt und der relative Anteil der VLP im Retentat zunimmt. Eine Konzentrierung ist bei vielen Aufreinigungsverfahren von Vorteil, weil damit Folgeschritte wie z.B. eine säulenchromatographische Aufreinigung effizienter und kostengünstiger durchzuführen sind.

Weiterhin wird dieser Aufreinigungsschritt dazu genutzt, die Lösungsbedingungen der VLP-enthaltenden Zusammensetzung zu verändern, indem z.B. der Puffer ausgetauscht wird (sog. Umpufferung). Dieser Vorgang wird auch als Diafiltration bezeichnet.

Das erfindungsgemäße Aufreinigungsverfahren weist außerdem wenigstens einen der folgenden Vorteile auf:
- Es stellt ein schonendes Aufreinigungsverfahren dar, das die VLP möglichst in ihrer nativen Form belässt, die für ihre Aktivität essentiell ist. Bei Verfahren nach dem Stand der Technik kommt es hingegen z.T. zu Veränderungen der VLP-Oberfläche (z.B. hinsichtlich Ladungsveränderungen), die sich nachteilig auf die nachfolgende Verwendung oder Verarbeitung der VLP auswirken können.
- Es kann die Verwendung organischer Lösungsmittel vermieden werden, was andernfalls die Gefahr eine Proteindenaturierung mit sich bringen kann.
- Das ionische Milieu und der pH-Wert der VLP-haltigen Zusammensetzung können bei Bedarf erhalten bleiben.
- Der erfindungsgemäße Filtrationsschritt ist schnell und kostengünstig. Außerdem ist er effizient und kann gleichzeitig zur Aufreinigung, Aufkonzentration und/oder Umpufferung verwendet werden.
- Es kann bei niedrigen Temperaturen durchgeführt werden, so z.B. in einem Kühlraum.

Der besondere Vorteil des erfindungsgemäßen Verfahrens ist jedoch, dass durch nur einen Schritt eine erhebliche Verbesserung der Reinheit der VLP in einer höchst komplexen und stark verunreinigten Zusammensetzung erreichbar wird. Dies gilt auch für so komplexe Zusammensetzungen wie einen Zellkulturüberstand. So kann die Reinheit der VLP-haltigen Zusammensetzung nach der erfindungsgemäßen Filtration mindestens 70%, vorteilhafterweise mindestens 75% oder mindestens 80% betragen. Die Reinheit kann durch weitere Aufreinigungsprozesse noch erhöht werden.

### Definitionen

Die in der Beschreibung und den Ansprüchen benutzten Begriffe, insofern nicht anders spezifiziert, besitzen die im Folgenden definierte Bedeutung:
Der Begriff "Virus-ähnlicher Partikel (VLP)" im Sinne der Erfindung bezieht sich auf eine partikuläre Struktur, in der mehrere Proteine aggregiert vorliegen, wobei sie in bevorzugter Weise einen Hohlraum umschließen. Zumindest ein Teil der Struktur bildenden Proteine ist identisch zu oder abgeleitet von viralen Strukturproteinen (Kapsidproteinen), insbesondere von Viren aus der Familie der Papoviridae. Diese umfasst die Familie der Papillomaviridae als auch die Familie der Polyomaviridae. Die VLPs können aber auch anderen Virusfamilien entstammen, wie z.B. der Familie der Parvoviridae, Flavoviridae und Retroviridae. Die erfindungsgemäßen VLP sind aus dem Strukturprotein VP1 des humanen JC-Virus aufgebaut.

Vorzugsweise wird ein VLP von 60, 72, 120, 180, 240, 300, 360 und mehr als 360 viralen Strukturproteinen gebildet und kann eine tubuläre oder sphärische Struktur aufweisen. Ein VLP aus 360 Strukturproteinen ist üblicherweise aus 72 Pentameren aufgebaut, die jeweils durch fünf monomere Strukturproteine gebildet werden. Die Aggregation der Strukturproteine und Pentamere kann durch eine nicht-kovalente oder eine kovalente Bindung der Proteine erfolgen. Bei einer kovalenten Bindung ist eine Ausbildung von Disulfidbrücken bevorzugt.

Ein VLP kann entweder aus einer Vielzahl nur eines Strukturproteins aufgebaut sein, oder aber verschiedene Strukturproteine umfassen. Bevorzugt ist das Vorhandensein nur eines Strukturproteins, nämlich des VP1.

Offenbart werden Strukturproteine der VLP, insbesondere das VP1 (aber auch VP2 und/oder VP3), welche identisch sein mit oder abgeleitet sein können von den Strukturproteinen z.B. der folgenden Viren aus der Familie der Polyomaviridae: Meerkatzen-Polyomavirus (AGMPyV), Pavian-Polyomavirus 2 (BPyV-2), Humanes Polyomavirus 1 (BK-Virus, BKV oder BKPyV), Humanes Polyomavirus 2 (JC-Virus, JCV oder JCPyV), Bovines Polyomavirus (BPyV), Wellensittich-Polyomavirus (Polyomavirus der Nestlingskrankheit der Wellensittiche, BFPyV), Hamster-Polyomavirus (HaPyV), Murines Pneumotropes Virus (MPtV), Murines Polyomavirus (MPyV), Kaninchen-Polyomavirus (Rabbit kidney vacuolating virus, RKV), Simian-Virus 12 (SV-12), Simian-Virus 40 (SV-40), Krähen-Polyomavirus, Hämorrhagisches Polyomavirus der Gänse (GHPV), Merkel-Cell-Polyomavirus, Schimpansen-Polyomavirus, Finken-Polyomavirus und KI-Polyomavirus (KIV).

Offenbart werden VLP, welche aber auch den Strukturproteinen der Viren, vorzugsweise dem L1 (aber auch dem L2) aus der Familie der Papillomaviridae entsprechen können oder davon abgeleitet sind, so nämlich beispielsweise von den folgenden Virengattungen: Alphapapillomavirus, Betapapillomavirus, Gammapapillomavirus, Deltapapillomavirus, Epsilonpapillomavirus, Zetapapillomavirus, Etapapillomavirus, Thetapapillomavirus, lotapapillomavirus, Kappapapillomavirus, Lambdapapillomavirus, Mupapillomavirus, Nupapillomavirus, Xipapillomavirus, Omikronpapillomavirus, Pipapillomavirus, Trichosurusvulpecula-Papillomvirus, und Opossum-Papillomvirus.

Das erfindungsgemäße VLP kann außerdem ein oder mehrere zusätzliche heterologe Proteine im Kapsid aufweisen, d.h. Proteine, die nicht mit einem Protein eines Virus der Familie der Papoviridae identisch oder ähnlich sind. Als heterologes Protein eignen sich prinzipiell alle Proteine, die in das Kapsid inkorporiert werden können oder an das Kapsid anbinden und die Assemblierung des VLP nicht maßgeblich beeinträchtigen.

Eine "VLP-enthaltende oder VLP-haltige Zusammensetzung" ist eine jegliche Zusammensetzung, die VLP enthält, vorzugsweise eine Flüssigkeit. Hierbei umfasst der Begriff "Flüssigkeit" in Abgrenzung zu einem Festkörper alle fließfähigen Zusammensetzungen, also auch hochviskose, ölige oder auch bituminöse Flüssigkeiten. Die Zusammensetzung kann ein- oder mehrphasig sein. Die VLP können gelöst vorliegen, aber auch ggf. unter Aggregatbildung als dispergierte oder suspendierte Teilchen.

Gemäß der Erfindung bezieht sich der Begriff "Filtermedium" auf jedes Medium, das eine Fest/Flüssig-Trennung erlaubt. Vorzugsweise liegt es als poröse Matrix vor, die mindestens zwei Kompartimente trennt und den Durchtritt einzelner Substanzen aus einem Kompartiment in mindestens ein zweites Kompartiment erlaubt. Ein jegliches Verfahren, das unter Verwendung eines Filtermediums durchgeführt wird, ist im Rahmen der vorliegenden Erfindung als "Filtration" aufzufassen.

Eine "Membran" im Sinne der Erfindung ist eine meist flächig ausgedehnte Struktur, die Poren aufweist. Vorzugsweise ist die Membran flexibel und besteht aus einem Polymer oder Polymergemisch. Als Polymer werden nach der Erfindung insbesondere Polyethersulfon oder Cellulose (insbesondere regenerierte Cellulose) verwendet.

Erfindungsgemäß gibt die "Ausschlußgrenze" (auch "Trenngrenze") die Rückhalterate der Filtration an. Sie wird üblicherweise auf die Molmasse bezogen und im Dalton angegeben (NMWC, englisch: Nominal Molecular Weight Cut-Off, auch MWCO, Molecular Weight Cut Off). Sie wird definiert als die minimale Molekülmasse eines Moleküls, welches durch das Filtermedium zurückgehalten wird.

Ein "Pentamer" im Rahmen der Erfindung ist eine Struktur, die durch fünf Polypeptiduntereinheiten gebildet wird. Die Bindung zwischen den einzelnen Polypeptiduntereinheiten kann durch nicht-kovalente oder kovalente Bindung erfolgen. Die fünf Untereinheiten bilden häufig eine ringförmige Struktur mit pentagonaler Symmetrie. Dabei geht in der Regel jede Untereinheit mit jeweils zwei benachbarten Untereinheiten eine Interaktion ein.

Unter "Dissoziation" ist im Rahmen der Erfindung der Vorgang zu verstehen, bei dem die Integrität des VLP so beeinträchtigt wird, dass der vom VLP vorzugsweise umschlossene Raum in Verbindung mit dem das VLP umgebende Außenmedium gelangt und/oder Kapsidproteine von dem VLP abgetrennt werden. Typischerweise wird dies durch die Abspaltung einiger Polypeptide oder Proteine, die das VLP bilden, erreicht. Hierbei kann das VLP auch gänzlich in seine Untereinheiten, wie z.B. VP1- oder L1-Pentamere zerfallen.

Gemäß der Erfindung ist eine "Reassoziation" eine teilweise oder vollständige Wiederherstellung eines VLP ausgehend von einer vorherigen VLP-Dissoziation.

Die Abkürzung "GMP" ist die Kurzbezeichnung für "Good Manufacturing Practice Regulations". In diesen Grundregeln der Weltgesundheitsorganisation sind die für die Herstellung von Arznei- und Lebensmitteln anerkannten Regeln und Maßnahmen zusammengestellt, die aufgrund des aktuellen Stands der Technik eine für den Verbraucher (Patienten oder Konsumenten) sichere Produktion garantieren. Die GMP-Regeln fordern neben einer angemessenen Ausstattung an Personal, Räumen und Maschinen ein System von Sicherheitsmassnahmen, das sich von der Eingangskontrolle bis zur Ausgangskontrolle über den ganzen Herstellprozess erstreckt.

Unter "Verunreinigungen" sind diejenigen Bestandteile der VLP-enthaltenden Zusammensetzung zu verstehen, die in der Zusammensetzung unerwünscht sind und daher möglichst abgereichert werden sollen. Das können z.B. Salze, niedermolekulare oder aber auch makromolekulare Verbindungen sein. Die Abreinigung der Vereinreinigungen führen zu einer Erhöhung der Reinheit des gewünschten Stoffes, also zu der Erhöhung des Stoffmengenanteils des gewünschten Stoffes (hier VLP) zum gesamten Stoffgemisch.
Die Begriffe "Anionenaustauscher" oder "Anionenaustauschermatrix" sind synonym und beziehen sich beide auf natürliche oder künstliche Substanzen, die Anionen binden können und diese gegen Anionen aus einem umgebenden Medium austauschen können. Ein Anionenaustauscher trägt positive Ionen und tauscht negativ geladene Gegenionen aus.

"Starke Anionenaustauscher" im Sinne der Erfindung tragen quarternäre Ammoniumgruppen vom Typ I: 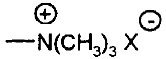 oder vom Typ II: 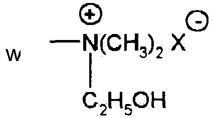 das ausgewählt ist aus der Gruppe bestehend aus Hydroxyl, Chlorid, Sulfat, Bromid, Jodid, Fluorid, Sulfid, Hydrogensulfat, Hydrogensulfid, Phosphat, Diphosphat, Monophosphat, Carbonat, Hydrogencarbonat, Citrat, Tartrat und Phthalat. Eine kommerziell erhältliche Säule mit einer starken Anionenaustauschermatrix ist beispielsweise die Mono Q-Säule der Firma GE Healthcare (München, BRD).

"Schwache Anionenaustauscher" im Sinne der Erfindung tragen tertiäre oder sekundäre Amingruppen als funktionelle Gruppen, wie beispielsweise Diethylaminoethyl (DEAE)-Gruppen. Eine kommerziell erhältliche schwache DEAE-Anionenaustauschermatrix ist beispielsweise der "Sartobind Q membrane adsorber" der Firma Sartorius (Göttingen, BRD).

Der Begriff "Ultrafiltration" bezieht sich auf ein Verfahren, bei dem eine Flüssigkeit (typischerweise unter Druck) in Kontakt mit einem Filtermedium, insbesondere einer semipermeablen Membran gebracht wird. Der Druck kann durch Ausüben eines Drucks auf die oberhalb der Filtermembran sich befindenden Flüssigkeit oder durch Zentrifugation der Filtereinheit angelegt werden. Alternativ kann durch das Anlegen eines Unterdrucks unterhalb der Filtermembran der Durchtritt der Lösung beschleunigt werden. Die Membran enthält Poren definierter Größe, so dass Moleküle oder Komplexe, die klein genug sind, um porengängig zu sein, durch die Membran zur gegenüberliegende Seite wandern, wohingegen Moleküle oder Komplexe, die zu groß sind, um die Poren passieren zu können, auf der Auftragsseite der Membran verbleiben. Ultrafiltrationsmembranen bestehen typischerweise aus Polymeren oder Polymergemischen und sind auf eine spezifische Molekulargewichts-Trenngrenze (Ausschlußgrenze) ausgelegt.

Der Begriff "Diafiltration" bezieht sich auf eine Form der Ultrafiltration, die die Eigenschaften der Dialyse mit der der Ultrafiltration verbindet. Die Zugabe eines Lösungsmittels zu dem Retentat der Filtration erlaubt einen Wechsel oder eine Verdünnung des originären Lösungsmittels (z.B. "Umpufferung").

Ein "Zellkulturüberstand" im Rahmen der Erfindung ist der aus einer Zellkultur stammende Teil der Zellkultur, der im Wesentlichen frei von VLP sezernierenden Zellen ist. In einer bevorzugten Ausführungsform der Erfindung sind in dem Zellkulturüberstand weniger als 5%, bevorzugt weniger als 2%, insbesondere weniger als 1% VLP sezernierende Zellen enthalten. Der Zellkulturüberstand enthält üblicherweise einen hohen Anteil von Verunreinigungen (z.B. Zelltrümmern, Proteinen, oder Fragmenten davon). Der Zellkulturüberstand kann behandelt werden, z.B. chemisch oder physikalisch. Der Überstand kann auch zentrifugiert werden. Er kann aber auch unbehandelt, d.h. z.B. ohne Zentrifugation erfindungsgemäß aufgereinigt werden. Bevorzugt wird er zentrifugiert, aber nicht chemisch oder enzymatisch behandelt. Der Begriff Zellkulturüberstand setzt nach der Erfindung voraus, dass eine Chromatographie des geernteten Ausgangsmediums nicht stattgefunden hat.

Als "Chromatographie" wird ein Verfahren bezeichnet, das die Auftrennung eines Stoffgemisches durch unterschiedliche Verteilung seiner Einzelbestandteile zwischen einer stationären und einer mobilen Phase erlaubt. Eine Zentrifugation ist in diesem Sinne keine Chromatographie.

Als "reversed phase"-Chromatograpie wird ein Verfahren bezeichnet, bei dem die stationäre Phase eine unpolare Phase und die mobile Phase eine polare Phase darstellt. Jede inerte unpolare Substanz, die sich als Säule packen lässt, kann für die "reversed phase"-Chromatograpie verwendet warden. Beispiele für stationäre Phasen sind eine an Silica gebundener Octadecyl-Rest (C18), Octylrest (C8), eine Caynogruppen oder Phenylgruppen tragendes Silica-Material oder reines Silica-Material. Als unpolare Phase. In der HPLC wird hierbei oft eine Gradienteneluierung angewandt, wobei die Zusammensetzung des Lösungsmittels langsam geändert wird (z. B. von 80 % auf 20 % Wasseranteil). So treten unpolare Bestandteile sehr spät und polare Bestandteile sehr früh aus der Säule aus und man kann sie somit voneinander trennen.

Ein "Zellaufschluß" im Rahmen der Erfindung ist eine Zusammensetzung, die sich durch das Aufschließen von Zellen ergibt. Der Aufschluß als Zerstörung der Zellintegrität kann durch chemische Methoden (z.B. Detergentien), biologische Methoden (z.B. enzymatische Behandlung) und/oder physikalische Methoden (z.B. Ultraschallbehandlung, Scherkräfte) erreicht werden. In diesem Sinne kann ein Zellaufschluß einen Zellkulturüberstand darstellen.

Der Begriff "zellfreies in vitro-Translationsgemisch" bezieht sich auf ein experimentelles Verfahren der Molekularbiologie, mit dessen Hilfe aus Zellen isolierte oder mittels In-vitro-Transkription erzeugte mRNA-Moleküle im Reaktionsgefäß zur Proteinbiosynthese verwendet werden (Translation). Dabei sind so genannte In-vitro-Translationssysteme hilfreich, die die nötigen Enzyme und tRNA-Moleküle sowie Aminosäuren enthalten, sodass es nach Zugabe der mRNA zur Proteinsynthese kommt. Häufig wird dem Reaktionsgemisch mit dem Schwefelisotop ³⁵S markiertes Methionin zugesetzt, um die Translationsprodukte nachweisen zu können. Gängige zellfreie Systeme sind Weizenkeimextrakt und Retikulocytenlysat.

### Die Erfindung im Einzelnen

Nach der Erfindung wird eine VLP haltige Zusammensetzung über ein Filtermedium mit einer Ausschlußgrenze von mehr als 30 kDa bis 1500 kDa filtriert. Es kann bevorzugt sein, ein Filtermedium mit einer Ausschlußgrenze von mindestens 40 kDa zu verwenden. In einer besonderen Ausführungsform liegt die Ausschlußgrenze des Filtermediums bei 80 bis 1500 kDa; insbesondere bevorzugt ist eine Ausschlußgrenze von etwa 100 kDa.

Ein Filtermedium, das eine Ausschlußgrenze in dem vorgenannten Bereich besitzt, erlaubt die effiziente Abtrennung auch höhermolekularer Verunreinigungen aus der VLP haltigen Zusammensetzung unter Minimierung des Ausbeuteverlusts an VLP.

Bei der VLP haltigen Zusammensetzung handelt es sich um den Zellkulturüberstand aus der Zellkultur der VLP sezernierenden Zellen. Dieser Zellkulturüberstand kann unmittelbar der Zellkultur entnommen und dann erfindungsgemäß filtriert werden. In einer Ausführungsform der Erfindung ist es jedoch auch möglich, dass der Zellkulturüberstand zuvor behandelt wird, z.B. chemisch, enzymatisch oder thermisch. Diese Behandlung kann unter Umständen die erfindungsgemäße Filtration erleichtern, ohne aus der Erfindung selber herauszuführen.

Das nach der Erfindung einsetzbare Filtermedium kann aus einem Polymer oder einem Polymergemisch bestehen. Bevorzugt ist hierbei ein Polymer oder Polymergemisch ausgewählt aus der Gruppe umfassend Cellulose, Polyethersulfon (PES), Cellulosetriacetat (CTA), Celluloseacetat, Cellulosenitrat, Polyacrylnitril (PAN) Polyamid, Polycarbonat und Polytetrafluorethylen (PTFE). Diese Polymere bilden stabile Filtermembranen aus, die inert sind, nicht zur Proteinaggregation neigen und mit Hohlräumen von kontrolliertem Durchmesser versehen werden können.

In einer Ausführungsform der Erfindung wird beim Filtrieren zwischen dem filtratseitigen und dem retentatseitigen Kompartiment eine Druckdifferenz zwischen 0,5 bar und 10 bar, bevorzugt zwischen 0,5 und 5 bar und besonders bevorzugt zwischen 0,5 bar und 3 bar aufgebaut.

Dem Fachmann stehen zahlreiche Methoden zur Verfügung, um einen entsprechenden Druck aufzubauen und zu regulieren. So kann durch Zentrifugation der Filtereinheit ein entsprechend der Zentrifugalbeschleunigung g entstehender Druck auf das Retentat ausgeübt werden. Weiterhin kann durch Anlegen eines retentatseitigen Überdrucks z.B. mittels einer Pumpe oder eines Stempels ein Druck aufgebaut werden. Alternativ kann die Druckdifferenz auch durch Anlegen eines filtratseitigen Unterdrucks, z.B. durch eine Vakuumpumpe aufgebaut werden. Die Druckdifferenz kann über die Zeit der Aufreinigung konstant gehalten werden, es kann aber auch ein zeitlich variierende Druckdifferenz aufgebaut werden, die z.B. als Filtrationsprogramm unterschiedliche Phasen mit jeweiliger zeitlicher Dauer und unterschiedlichen Druck beinhaltet.

In einer bevorzugten Ausführungsform kann die Filtration als sog. "Cross-Flow-Filtration" (auch "Tangential-Flow-" oder "Querstromfiltration" genannt) durchgeführt werden. Hierbei kann eine Querströmung mit einer hohen Geschwindigkeit von z.B. etwa 2,5 bis 3 m/s erzeugt werden, die entlang einer Membran oder eines Filtermediums fließt. Durch die hohe Geschwindigkeit wird vermieden, dass sich ein Filterkuchen (Deckschicht oder Fouling) aus den abzutrennenden Feststoffpartikeln auf der Membran aufbauen kann.

In einer alternativen Ausführungsform der Erfindung kann die Filtration aber auch im Dead-End-Verfahren durchgeführt werden. Bei der Dead-End-Filtration wird ein Feedstrom, um die Kompaktierung der zurückgehaltenen Stoffe zu minimieren, mit möglichst niedrigem Druck gegen die Membran gepumpt. Durch den permanenten Abfluss des Permeats reichert sich ein Filterkuchen (Deckschicht oder Fouling) oder ein Konzentrationsgradient (Konzentrationspolarisation) aus den abzutrennenden Proteinpartikeln auf der Membran an. Der Filterkuchen erhöht den Filtrationswiderstand und damit den Druckverlust über die Membran. Er muss, je nach Feedzusammensetzung, in regelmäßigen Intervallen durch Rückspülung (Zurückpumpen von bereits abgetrenntem Medium) und chemischen Reinigungen entfernt und das Filterelement somit regeneriert werden.

Gemäß der Erfindung kann das Filtermedium verschiedene Geometrien aufweisen, so z.B. kann es als spiralförmig gewickelte Membran, röhrenförmige Membran oder Hohlfasermembran ausgebildet sein. Die unterschiedliche Filtergeometrie trägt den Prozessbedingungen in Hinsicht auf Lösungsvolumen, Konzentration/Menge an VLP bzw. Verunreinigungen oder angelegte Druckdifferenz Rechnung.

Bei dem offenbarten Aufreinigungsverfahren können neben dem oben bereits erwähnten Zellkulturüberstand auch andere VLP-enthaltende Zusammensetzungen eingesetzt werden. Mögliche Medien nach der Offenbarung sind daher ausgewählt aus der Gruppe folgender Medien:
(a) Zellkulturüberstand aus der Kultivierung von VLP exprimierenden Zellen;
(b) Zellkulturüberstand nach (a) nach Aufreinigung über Zentrifugation und/oder Dialyse;
(c) Zellaufschluß von (a);
(d) Zellaufschluß (nach) nach Aufreinigung über Zentrifugation und/oder Dialyse;
(e) Zellfreies *in vitro*-Translationsgemisch; oder
(f) Zellfreies *in vitro*-Translationsgemisch nach Aufreinigung über Zentrifugation und/oder Dialyse.

In einer weiteren Ausführungsform der Erfindung wird neben der Aufreinigung auch eine Aufkonzentration der VLP-enthaltenden Zusammensetzung erreicht. Dies geschieht vorzugsweise dadurch, dass das während der Filtration abnehmende Retentatvolumen nicht oder nur teilweise aufgefüllt wird.

Erfindungsgemäß wird neben der Aufreinigung auch eine Umpufferung der VLP-enthaltenden Zusammensetzung erreicht. Dieser Pufferaustausch geschieht vorzugsweise dadurch, dass das während der Filtration abnehmende Retentatvolumen durch einen Puffer mit einer bezüglich des Ausgangspuffers abweichenden Zusammensetzung ersetzt wird.

Hierbei wird in einer bevorzugten Ausführungsform der pH-Wert abgesenkt, wobei ein pH-Wert nahe des pl-Werts der Kapsidproteine vorteilhaft ist. Bei den aus VP1 bestehenden VLPs ist dies ein pH-Wert zwischen 5,0 und 8,0. Dieser erniedrigte pH-Wert resultiert in einer reduzierten Stabilität der VLPs, die somit bei einem nachfolgenden Dissociationsschritt leichter dissoziiert werden können, und so beispielsweise geringere Mengen an Disulfidbrücken-spaltendem Reduktionsmittel und Ca²⁺-Ionen bindendem Komplexbildner verwendet werden müssen.

In einer Ausführungsform der Erfindung wird die mit dem erfinderischen Verfahren aufgereinigte VLP-enthaltende Zusammensetzung zusätzlich chromatographisch, vorzugsweise über eine Anionenaustausch-Chromatographie aufgereinigt.

In bevorzugter Weise erfolgt die zusätzliche Aufreinigung durch Auftrennung mittels eines (insbesondere schwachen) Anionenaustauschers. Dieser enthält als funktionale Gruppen primäre, sekundäre oder tertiäre Amingruppen, wobei Diethylaminoethyl (DEAE)-Gruppen bevorzugt sind. In einer bevorzugten Ausführungsform der Erfindung besteht die Matrix dieses Anionenaustauschers aus DEAE-Sepharose.

Die Elution der VLP bzw. VP1-Pentamere von diesem Anionenaustauscher kann erfindungsgemäß durch eine NaCI-haltige Lösung erfolgen, die bevorzugt NaCl in einer Konzentration von 150 mM bis 750 mM, besonders bevorzugt 300 mM NaCl enthält.

In einer weiteren Ausführungsform der Erfindung umfasst die zusätzliche Aufreinigung der VLP mittels eines Anionenaustauschers die folgenden Schritte:
(a) Bereitstellen der mittels Ultrafiltration aufgereinigten VLP-enthaltenden Zusammensetzung;
(b) Inkontaktbringen der VLP-enthaltenen Zusammensetzung mit einem Anionenaustauscher unter Bedingungen, die eine Bindung der VLP an den Anionenaustauscher ermöglichen;
(c) optionales Waschen des Anionenaustauschers;
(d) Elution der gebundenen VLP;
(e) optionale Dialyse der VLP gegen eine wässrige Lösung.

Vorzugsweise wird dazu ein schwacher Anionenaustauscher verwendet.

In einer weitere Ausführungsform der Erfindung wird die erfindungsgemäß aufgereinigte VLP-enthaltende Zusammensetzung hierbei mit einem starkem Anionenaustauscher aufgereinigt, der quartäre Ammoniumgruppen vom Typ I oder Typ II enthält: 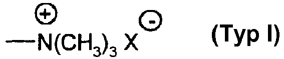 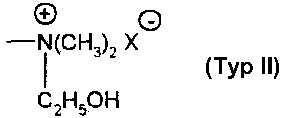 wobei X ein Anion ist ausgewählt aus der Gruppe bestehend aus Hydroxyl, Chlorid, Sulfat, Bromid, Jodid, Fluorid, Sulfid, Hydrogensulfat, Hydrogensulfid, Phosphat, Diphosphat, Monophosphat, Carbonat, Hydrogencarbonat, Citrat, Tartrat oder Phthalat.

In einem bevorzugten Aspekt der Erfindung wird hierbei als starker Anionenaustauscher eine Q-Sepharose-Matrix oder eine MonoQ-Säule verwendet.

Die Elution der VLP von dem starken Anionenaustauscher kann durch eine oder mehrere NaCl-enthaltende wässrige Lösungen erfolgen. Hierbei kann die Variation der NaCl-Konzentration durch einen linear ansteigenden Gradienten oder einen Stufengradienten erfolgen.
Als linearer NaCI-Gradient wird ein Gradient bevorzugt, bei dem die NaCI-Konzentration des Elutionspuffers von initial 100 mM bis auf 1 M NaCl ansteigt. Als NaCI-Stufengradient ist ein dreistufiger oder ein vierstufiger Gradient bevorzugt.

Der dreistufige NaCI-Stufengradient kann bevorzugt aus den folgenden Schritten bestehen:
(i) 50 mM bis 150 mM NaCl,
(ii) 200 mM bis 400 mM NaCl,
(iii) 1 M bis 2 M NaCl; oder

Der vierstufige NaCI-Stufengradient kann bevorzugt aus den folgenden Schritten bestehen:
(i) 50 mM bis 150 mM NaCl,
(ii) 200 mM bis 400 mM NaCl,
(iii) 500 mM bis 800 mM NaCl
(iv) 1 M bis 2 M NaCl.

In einer weiteren Ausführungsform der Erfindung wird die mit dem erfinderischen Verfahren aufgereinigte VLP-enthaltende Zusammensetzung zusätzlich mittels Gelfitration oder mittels keramischer Hydroxyapatit-Säulenchromatographie aufgereinigt. Diese Verfahren erlauben eine Abtrennung der VLP von Kapsidmomomeren oder Kapsidoligomeren wie bsp. Pentameren.

In einer Ausführungsform der Erfindung werden die mit dem erfindungsgemäßen Verfahren aufgereinigten VLP durch ein Verfahren gemäß der folgenden Schritte aufgereinigt:
(a) Dissoziation der VLP;
(b) Aufreinigung der dissoziierten VLP;
(c) Reassoziation der dissoziierten VLP.

Die Dissoziation der VLP erfolgt vorzugsweise in Gegenwart eines Disulfidbrücken-spaltenden Reduktionsmittels und eines Ca²⁺-Ionen bindende Komplexbildners.

In einem Aspekt der Erfindung werden als Disulfidbrücken-spaltende Reduktionsmittel schwefelhaltige Verbindungen verwendet, bevorzugt ausgewählt aus der Gruppe bestehend aus 2-Mercaptoethanol (2-ME), Dithiothreitol (DTT), Dithioerythrol (DTE), Glutathion, Tris(2-carboxyethyl)phosphin (TCEP), Monothioglycerol und besonders bevorzugt DTT.

In Fällen, bei denen eine Toxizität besonders kritisch ist, sind die Disulfidbrücken-spaltenden Reduktionsmittel 2-Mercaptoethanol (2-ME) und Monothioglycerol zu bevorzugen. 2-ME kann aufgrund seiner Leichtflüchtigkeit einfach entfernt werden, Monothioglycerol hingegen ist auch für einen anschließenden Einsatz in der Zellkultur geeignet.

Die schwefelhaltigen Reduktionsmittel stellen schwache Reduktionsmittel dar, deren Redoxpotential aber ausreicht, um die Disulfidbrücken zu den freien Cystein-Resten zu reduzieren. Die Verwendung solch schwacher Reduktionsmittel hat den Vorteil, dass andere funktionelle Gruppen der VLP nicht reduziert werden und so die Integrität und native Beschaffenheit der VLP im Wesentlichen erhalten bleiben. Erfindungsgemäß können auch andere Reduktionsmittel eingesetzt werden, deren Redoxpotential in den Bereich der oben genannten schwefelhaltigen Reduktionsmittel fällt.

In einem weiteren Aspekt der Erfindung stellt der bei der Dissoziation verwendete, Ca²⁺-Ionen bindende Komplexbildner ein chelatisierendes Agens dar, bevorzugt ausgewählt aus der Gruppe bestehend aus Zitronensäure, Ethylendiamintetraessigsäure (EDTA), Ethylenglykoltetraessigsäure (EGTA) oder den entsprechenden Salzen dieser Säuren und besonders bevorzugt EGTA.

In einer Ausführungsform der Erfindung werden die dissoziierten VLP, die bevorzugt als Kapsidproteine vorliegen - besonders bevorzugt als VP1- oder L1-Proteine - zusätzlich chromatographisch, vorzugsweise über eine Anionenaustausch-Chromatographie oder "reversed phase" Chromatographie aufgereinigt.

In einer bevorzugten Ausführungsform wird hierbei ein starker Anionenaustauscher verwendet, der quartäre Ammoniumgruppen vom Typ I oder Typ II enthält: 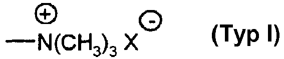 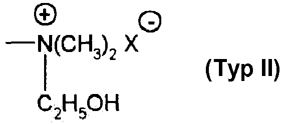 wobei X ein Anion ist ausgewählt aus der Gruppe bestehend aus Hydroxyl, Chlorid, Sulfat, Bromid, Jodid, Fluorid, Sulfid, Hydrogensulfat, Hydrogensulfid, Phosphat, Diphosphat, Monophosphat, Carbonat, Hydrogencarbonat, Citrat, Tartrat oder Phthalat.

In einem bevorzugten Aspekt der Erfindung wird hierbei als starker Anionenaustauscher eine Q-Sepharose-Matrix oder eine MonoQ-Säule verwendet.

Die Elution der dissoziierten VLP (bzw. der Strukturproteine davon) von dem starken Anionenaustauscher kann erfindungsgemäß durch eine oder mehrere NaCl-enthaltende wässrige Lösungen erfolgen. Hierbei kann die Variation der NaCI-Konzentration durch einen linear ansteigenden Gradienten oder einen Stufengradienten erfolgen.
Als linearer NaCI-Gradient wird ein Gradient bevorzugt, bei dem die NaCI-Konzentration des Elutionspuffers von initial 100 mM bis auf 1 M NaCl ansteigt. Als NaCI-Stufengradient ist ein dreistufiger oder ein vierstufiger Gradient bevorzugt.

Der dreistufige NaCI-Stufengradient kann bevorzugt aus den folgenden Schritten bestehen:
(i) 50 mM bis 150 mM NaCl,
(ii) 200 mM bis 400 mM NaCl,
(iii) 1 M bis 2 M NaCl; oder

Der vierstufige NaCI-Stufengradient kann bevorzugt aus den folgenden Schritten bestehen:
(i) 50 mM bis 150 mM NaCl,
(ii) 200 mM bis 400 mM NaCl,
(iii) 500 mM bis 800 mM NaCl
(iv) 1 M bis 2 M NaCl.

In einer weiteren Ausführungsform der Erfindung umfasst die Aufreinigung der dissoziierten VLP mittels eines Anionenaustauschers die folgenden Schritte:
(c) Inkontaktbringen der dissoziierten VLP-enthaltenen Zusammensetzung mit einem Anionenaustauscher unter Bedingungen, die eine Bindung der dissoziierten VLP an den Anionenaustauscher ermöglichen;
(d) optionales Waschen des Anionenaustauschers;
(e) Elution der gebundenen dissoziierten VLP;
(f) optionale Dialyse der dissoziierten VLP gegen eine wässrige Lösung.

Vorzugsweise wird dazu ein starker Anionenaustauscher verwendet.

Die Aufreinigung der dissoziierten VLP kann erfindungsgemäß auch durch einen schwachen Anionenaustauscher erfolgen. Dieser enthält als funktionale Gruppen primäre, sekundäre oder tertiäre Amingruppen, wobei Diethylaminoethyl (DEAE)-Gruppen bevorzugt sind. In einer bevorzugten Ausführungsform der Erfindung besteht die Matrix des schwachen Anionenaustauschers aus DEAE-Sepharose.

Die Elution der dissoziierten VLP von dem schwachen Anionenaustauscher erfolgt erfindungsgemäß durch eine NaCI-haltige Lösung, die bevorzugt NaCl in einer Konzentration von 150 mM bis 750 mM, besonders bevorzugt 300 mM NaCl enthält.
In einer weiteren Ausführungsform der Erfindung umfasst die Aufreinigung der dissoziierten VLP mittels eines Anionenaustauschers die folgenden Schritte:
(c) Inkontaktbringen der dissoziierten VLP-enthaltenen Zusammensetzung mit einem Anionenaustauscher unter Bedingungen, die eine Bindung der dissoziierten VLP an den Anionenaustauscher ermöglichen;
(d) optionales Waschen des Anionenaustauschers;
(e) Elution der gebundenen dissoziierten VLP;
(f) optionale Dialyse der dissoziierten VLP gegen eine wässrige Lösung.

Vorzugsweise wird dazu ein schwacher Anionenaustauscher verwendet.

In einer Ausführungsform der Erfindung werden die dissoziierten VLP über eine Reversed phase-Chromatographie aufgereinigt. Bevorzugt ist hier eine Aufreinigung über eine "High Performance Liquid Chromatography" (HPLC) unter Verwendung einer Reverse-Phase-Säule. Die erlaubt in kurzer Zeit eine Aufreinigung auch großer Mengen an dissoziierten VLP.

In einer weiteren Ausführungsform der Erfindung werden die dissoziierten VLP über eine Gel-Permeations-Chromatographie aufgereinigt. Dieses Verfahren ist bevorzugt in denen Fällen einzusetzen, bei denen modifizierte Kapsidproteine eingesetzt werden, die durch die Modifikation (insbesondere Insertionen oder Deletionen) ein andere Molekulargewicht erhalten und sich somit von den unmodifizierten Kapsidproteinen abtrennen lassen.

Die dissoziierten VLP können optional durch eine zusätzliche Dialyse aufgereinigt werden, um z.B. einen Austausch der Pufferbestandteile durchzuführen. Bevorzugt wird für die Dialyse ein Puffer verwendet, der bezüglich des Blutes osmomolar ist. Besonders bevorzugt ist hierbei die Verwendung von physiologischer Kochsalzlösung als Dialysepuffer.

Die Dialyse wird bevorzugt bei einer Temperatur unterhalb der Raumtemperatur durchgeführt, wobei eine Temperatur von 5 ± 3°C bevorzugt ist. Die Dauer der Dialyse beträgt zweckmäßigerweise mindestens 12 Stunden und ist bevorzugt länger als 16 Stunden. Die Dialyse kann bis zu 48 Stunden dauern.

Zur Lagerung der dissoziierten VLP ist es vorteilhaft, die dissoziierten VLP in einem Puffer mit niedrigem pH-Wert(5.0 bis 7.5) und mit erhöhter NaCI-Konzentration (250mM bis 500mM) aufzunehmen. Dadurch wird eine Aggregation verhindert. Die Lagerung erfolgt dann bei einer Temperatur von -80°C. Alternativ können die dissoziierten VLP auch durch Lyophilisierung in eine stabile Lagerform überführt werden.

In einer besonderen Ausführungsform kann das VLP im Inneren der Kapsidstruktur eine oder mehrere Substanzen enthalten. Solche Substanzen umfassen z.B. Makromoleküle, wie etwa Nukleinsäuren, d.h. RNA, DNA oder artifizielle modifizierte Nukleinsäuren, sowie Proteine und andere physiologisch aktive Stoffe, die natürlicher, synthetischer oder rekombinanter Art sein können. Beispiele für derartige physiologisch aktive Stoffe sind z.B. Lipide, Phospholipide, Peptide, Arzneimittel, Toxine etc.

Die Reassoziation der aufgereinigten und optional mit Wirkstoff beladenen VLP kann durch Dialyse mit einem Reassozationspuffer, der zweiwertige Kationen oder aber das einwertige Kation Rb⁺ enthält, erfolgen. Dieser Puffer enthält bevorzugt ein Kation ausgewählt aus der Gruppe bestehend aus Mg²⁺, Rb⁺ oder Zn²⁺ und besonders bevorzugt Ca²⁺. Der Reassozationspuffer enthält 0.1 mM bis 10 mM an Ca²⁺-Ionen, bevorzugt 0.5 mM bis 5 mM an Ca²⁺-Ionen und im Speziellen 1 mM CaCl₂.

In einer weiteren Ausführungsform der Erfindung werden die mit dem Wirkstoff beladenen VLP über eine Gel-Permeations-Chromatographie aufgereinigt. Dies erlaubt eine Abtrennung der Wirkstoffmoleküle von den mit Zielmolekülen beladenenen VLPs.

In einem weiteren Aspekt stellt die Erfindung ein Aufreinigungsverfahren bereit, das ausgehend von einem Zellkulturüberstand eine VLP-haltige Zusammensetzung mit einer Reinheit an VLP von mindestens als 80%, bevorzugt von mindestens 90%, insbesondere von mindestens 95% oder am meisten bevorzugt von mindestens 99% ergibt, und damit den bisherigen VLP-Aufreinigungsmethoden überlegen ist. Als Reinheit der VLP ist dabei der relative Anteil der VLP an dem gesamten Stoffgemisch als Ergebnis des Aufreinigungsverfahrens gemeint. Diese VLP Zusammensetzung ist frei von PEG und/oder Salzen und vorzugsweise PEG-frei.

Die VLP bestehen im Wesentlichen oder ausschließlich aus dem VP1-Kapsidprotein, das aus dem Kapsidprotein VP1 des humanen Polyomavirus JC abgeleitet (JCV) oder damit identisch ist. Die Sequenz des VP1 des humanen Polyomavirus JC ist in SEQ. ID. NO. 1 wiedergegeben.

Zur Herstellung von rekombinanten VP1 in Insektenzellen kann erfindungsgemäß eine Nukleinsäure verwendet werden, die für die Expression in diesen Zellen optimiert wurde. Beispielsweise kann die in SEQ. ID. NO. 2 gezeigte oder eine dazu komplementäre Sequenz, eine dieser Sequenz im Rahmen der Degeneration des genetischen Codes entsprechende Sequenz oder eine damit unter stringenten Bedingungen hybridisierende Sequenz verwendet werden. Dazu wird die Nukleinsäuresequenz oder ein diese Sequenz enthaltender rekombinanter Vektor in eine geeignete Wirtszelle eingebracht, die Wirtszelle unter Bedingungen kultiviert, bei denen eine Expression der Nukleinsäuresequenz erfolgt und das Protein aus der Zelle oder dem Zellüberstand isoliert. Stringente Hybridisierungsbedingungen sind vorzugsweise definiert gemäß Sambrook et al. (1989) Molecular Cloning A Laboratory Manual, Second Edition, Cold Spring Harbor Laboratory Press, und umfassen einen Waschschritt von 30 min in 0,1 x SSC, 0,5 % SDS bei 60°C und vorzugsweise 68°C.
Im Rahmen der vorliegenden Erfindung wird daher vorzugsweise ein VP1-Polypeptid eingesetzt, welches die Aminosäuresequenz nach SEQ. ID. NO. 3, eine hierzu mindestens 70 %, vorzugsweise mindestens 80 %, besonders bevorzugt mindestens 90 % und am meisten bevorzugt mindestens 95 % identische Aminosäuresequenz aufweist, wobei die Identität über den gesamten Bereich von SEQ ID No. 3 bestimmt wird.

In einer weiteren Ausführungsform wird ein VP1 Protein verwendet, bei welchem die Aminosäuresequenz im N-terminalen Bereich, z.B. im Bereich der 25 N-terminalen Aminosäuren verändert wurde. Dabei wird vorzugsweise ein heterologes Kernlokalisierungssignal in die Aminosäuresequenz des VP1 eingeführt. Bevorzugte Kernlokalisierungssignale enthalten die Aminosäuresequenz CPGAAPX1X2P, wobei X1 und X2 beliebige Aminosäuren und vorzugsweise jeweils K bedeuten, und z.B. auf den Kernlokalisierungssignalen von SV40 oder BKV basieren. Die Aminosäuresequenzen besonders bevorzugter Kernlokalisierungssignale sind in der EP 2 036 980 A1 offenbart, die hiermit vollumfänglich durch vorliegende Anmeldung mit aufgenommen ist.

Das durch rekombinante Expression hergestellte VP1 wird bevorzugt in eukaryoten Zellen und besonders bevorzugt in Insektenzellen exprimiert.

In einer weiteren Ausführungsform wird ein VP2 Protein (SEQ ID Nr. 6), ein VP3-Protein (SEQ ID Nr. 10), ein L1-Protein (SEQ ID Nr. 13) oder ein L2-Protein (SEQ ID Nr. 15) verwendet.

Für die rekombinante Expression können die Wildtyp-Nukleinsäuresequenzen verwenden werden, so beispielsweise für VP2 die SEQ ID Nr. 4, für VP3 die SEQ ID Nr. 8, für L1 die SEQ ID Nr. 12 und für L2 die SEQ ID Nr. 14.

In einer weiteren Ausführungsform werden die Nukleinsäuresequenzen für das entsprechende Expressionssystem kodonoptimiert. In einer besonderen Ausführungsform werden für die Expression in Insektenzellen die folgenden Nukleinsäuresequenzen für die Expression verwendet: SEQ ID Nr. 2 für ein VP1-Protein, SEQ ID Nr. 5 für ein VP2-Protein, SEQ ID Nr. 7 für ein VP2-HA Protein, SEQ ID Nr, 9 für ein VP3-HA Protein.

In einer besonderen Ausführungsform sind die jeweiligen Protein N-terminal oder C-terminal so verändert, dass die Verwendung als Vakzine verbessert wird. In einer besonderen Ausführungsform handelt es sich dabei um ein C-terminal lokalisiertes Peptid, das das Haemagluttinin-Epitop (als "HA" abgekürzt) umfasst. Beispiele für ein derart modifiziertes Protein sind das Protein VP2-HA (SEQ ID Nr. 7) und das Protein VP3-HA (SEQ ID Nr. 11).

In einer bevorzugten Ausführungsform werden zur rekombinanten Expression der Kapsidproteine VP1, VP2, VP2-HA, VP3-HA, L1 oder L2 und damit zur Bereitstellung von VLP Zellen aus *Spodoptera frugiperda* verwendet, wie beispielsweise die SF9-Zelllinie, die SF21-Zelllinie oder die SF158-Zelllinie. Es können auch andere Insektenzelllinien verwendet werden, wie z.B. Trichoplusia ni TN-368, IAL-TND1, Lymantria dispar IPLB-LdFB, Mamestra brassica IZD-MB0503, L. dispar IPLB-LdElta, Anticarsa gemmatalis UFL-AG286, Plodia interpunctella IAL-PID2, Plutella xylostella BCIRL-PxHNU3, T. ni BTI-TN5B1-4 (HiFive®), Manduca sexta MRRL-CH1, Heliothis virescens Linien: IPLB-HvT1, IPLB-HvE1A, IPLB-HvE6A, und die Diabrotica undecimpunctata Zelllinie IPLB-DU182A.

Die erfindungsgemäßen VLP können für diagnostische und therapeutische Zwecke eingesetzt werden, z.B. zur Diagnose, Prävention und Behandlung von Erkrankungen und Krankheitszuständen. Dazu können sie entweder therapeutisch aktive Substanzen enthalten, oder aber auch ohne eingeschlossene Substanzen verwendet werden.

In einer bevorzugten Ausführungsform handelt es sich bei der Erkrankung um eine Infektion, die durch den JC-Virus hervorgerufen wurde, wie etwa PML.

### BEISPIEL 1: Herstellung eines Baculovirus "Seed Strain"

### 1. Herstellung des rekombinanten Bacmids

Die kodonoptimierte Sequenz gemäß SEQ. ID. NO. 2 des VP1 Gens wurde in pFastBac1 Plasmide der Firma GENEART kloniert. Das Plasmid pFastBac1 wurde in DH5alfa E.coli Zellen transformiert, die Bakterienzellen vermehrt und die Bakterien als Glyzerol Stock bei - 80 °C gelagert. Das pFasBac-VP1 Plasmid wurde zur Vermehrung der Baculo-DNA in DH10Bac E.coli Zellen transformiert.

**1.1. Transformation der DH10Bac E.coli Bakterien**
Zur Revitalisierung der DH10Bac E.coli Bakterien werden die Max Efficiency DH10Bac E.coli Zellen aus dem Ultratiefkühlschrank (-80°C) entnommen, auf Eis aufgetaut und jeweils 100µl der Bakterien entnommen und in ein 10 ml Proberöhrchen transferiert.

**1.2.** Die DH10Bac E.coli Zellen werden mit 1ng (=1 µL) pFasBac-VP1 Plasmid vermischt und 30±3 min auf Eis inkubiert. Die Bakterien mit der Plasmid-DNA pFastBac1-VP1 transformiert.

**1.3.** Die rekombinante VP1-Baculo-DNA wird gemäß Standardmethoden isoliert.

### 2. Revitalisierung der kryokonservierten SF9 Zellen

Zur Revitalisierung der SF9 Zellen wird ein Kryoröhrchen der SF9 Zellen aus dem Stickstofflagertank entnommen und aufgetaut. Nach dem Ende des Auftauvorgangs werden die Zellen im 15 ml Proberöhrchen durch vorsichtiges dreimaliges Invertieren gleichmäßig verteilt und ein Aliquot für die Bestimmung der Zellzahl und Vitalität (Lebendzellzahl) entnommen.

Nach Abschluss des Auftauvorganges werden die Zellen in eine Zellkulturflasche geeigneter Größe (T75 oder T175) transferiert, so dass eine Zelldichte von 3 x 10⁵ vitaler Zellen pro Milliliter erreicht wird. Im Falle einer T75 wird die Zellsuspension auf ein Volumen von ≤30 ml, im Falle einer T175 Zellkulturflasche wird die Zellsuspension auf ein Volumen von >30 ml und ≤50 ml Vollmedium eingestellt und in die Zellkulturflasche übertragen. Zur Expansion werden die SF9 Zellen in einem Brutschrank bei einer Temperatur von 26 °C und einer Luftfeuchte von ≥ 80 % inkubiert.

### 3. Expansion der Zellen

Die Inkubation der Zellen im Brutschrank wird solange durchgeführt bis der Zellrasen eine Dichte von über 70 % aufweist. Dann werden die Zellen vom Boden des Zellkulturgefäßes abgelöst und auf eine größere bzw. mehrere Zellkulturflaschen verteilt. Hierzu werden die Zellen in der Zellkulturflasche mit PBS Puffer gespült und mit dem Zellschaber von ihrer Wachstumsoberfläche abgelöst. Zum gleichmäßigen Verteilen der Zellen im Vollmedium, wird das Kulturgefäß vorsichtig geschwenkt und anschließend ein Aliquot zur Zellzählung entnommen. Bei der erneuten Aussaat werden jeweils 1x10⁶ Zellen / Well in 3 ml Vollmedium ausgesät.

### 4. Transfektion der Insekten Zellen mit rekombinanten VP1-Baculo-DNA-Cellfectin Komplexen

16 bis 24 Stunden nach Aussaat der Zellen in 6 Well Platten wird das Zellkulturgefäß aus dem Brutschrank entnommen, das Vollmedium abgenommen und pro Well 1 ml vorgewärmtes, serumfreies Medium hinzugegeben.

Jetzt werden die DNA-Cellfectin Komplexe hergestellt. Hierzu werden 8 µl Cellfectin Reagenz mit 100 µl serumfreien Medium gemischt. 2 µg (= 2 µL) VP1-Baculo-DNA wird mit serumfreien Medium verdünnt. Der Cellfectin Ansatz wird mit dem VP1-Baculo-DNA Ansatz vereinigt und 30±2 min bei Raumtemperatur (20 ± 4 °C) inkubiert. 210µl des Cellfectin-DNA Transfektionsansatze werden in jedes Well der 6 Well Platte pipettiert und die Zellen im Brutschrank für 5 Stunden ± 15min bei 26 ± 1°C inkubiert. Anschließend wird das Medium mit dem Cellfectin-DNA Transfektionsansatz aus den einzelnen Wells der 6 Well Platte abgenommen und 2 ml/Well Vollmedium hinzugegeben. Die transfizierten Zellen werden 72 ± 1 Stunde bei 26 ± 1°C weiter inkubiert. Die rekombinanten Baculoviren werden während dieser Zeit ins Zellkulturmedium freigesetzt. Zur Ernte der rekombinanten Baculoviren wird das virushaltige Zellkulturmedium der transfizierten Zellen abgenommen und in ein 15 ml Proberöhrchen transferiert und für 10 min bei 2000xg bei 4°C zentrifugiert, um die enthaltenen Zelltrümmer der lysierten Zellen vom virenhaltigen Zellkulturmedium abzutrennen. Anschließend wird das virenhaltige Zellkulturmedium (der Überstand) in ein 2 ml Proberöhrchen transferiert und bei 5±3 °C bis zu seiner weiteren Verwendung gelagert. Die Baculoviren werden P1 genannt. 1 ml davon wird bei -80 ± 5 °C gelagert. Das restliche Volumen wird für die Herstellung des Seed Strains gelagert.

### 5. Herstellung des Baculovirus Seed Strains

### 5.1. Vorbereitung der Sf9 Insektenzellen für die Baculovirus Produktion

Die Zellen werden im Brutschrank inkubiert bis der Zellrasen eine Dichte von über 70 % aufweist. Dann werden die Zellen gemäß vom Boden des Zellkulturgefäßes abgelöst und auf eine größere Wachstumsoberfläche bzw. mehrere Zellkulturflaschen verteilt. Hierzu werden die Zellen in der Zellkulturflasche mit PBS Puffer gespült und mit dem Zellschaber von ihrer Wachstumsoberfläche abgelöst. Zum gleichmäßigen Verteilen der Zellen im Vollmedium, wird das Kulturgefäß vorsichtig geschwenkt und anschließend eine Probe zur Zellzahlbestimmung entnommen. Anhand der bestimmten Zellzahl werden jeweils 5x10⁶ Zellen pro T25 Zellkulturflasche in 5 ml Vollmedium ausgesät.

### 5.2. Infektion der SF9 Zellen mit den rekombinanten Baculoviren

16 bis 24 Stunden nach Aussaat der Zellen wird die Zellkulturflasche aus dem Brutschrank genommen und das Vollmedium visuell und mikroskopisch auf eine Trübung und damit auf eine mögliche Kontamination überprüft. Anschließend wird das Vollmedium abgenommen und 4 ml neues vorgewärmtes Vollmedium hinzu gegeben. Dazu wird 1ml vom P1 Baculovirus in Vollmedium gegeben. Während der folgenden Infektion werden die SF9 Zellen mit den rekombinanten Baculoviren bei 26 ± 2 °C für 3 (± 1) Tage im Brutschrank inkubiert.

### 5.3. Ernte der rekombinante Baculovirus

Zur Ernte der produzierten Baculoviren wird das Zellkulturmedium der infizierten Zellen abgenommen und in ein 15 bzw. 50 ml Proberöhrchen transferiert und für 10 min bei 2000xg bei 4 °C zentrifugiert, um die enthaltenen Zelltrümmer der lysierten Zellen vom virenhaltigen Medium abzutrennen. Anschließend wird das virenhaltige Zellkulturmedium (Überstand) bei 5 (± 3) °C gelagert.

Um den Titer der Viren zu quantifizieren wird die Baculovirus DNA aus 150µl virushaltiges Medium isoliert und mittels quantitativer PCR amplifiziert und quantifiziert. Die Baculoviren werden als P2 bezeichnet und bei -80 ± 5 °C gelagert.

### 6. Lagerung der rekombinanten Baculoviren

Von P1 und P2 werden jeweils zweimal 1 mL abgenommen und in beschriftete 2 ml Kryoröhrchen transferiert und als Rückstellproben bei - 80 (± 5) °C gelagert.

### BEISPIEL 2: Herstellung von VP1-VLP mittels Baculovirus vermittelter Expression in SF9-Zellen

### 1. Revitalisierung der kryokonservierten SF9 Zellen

Zur Revitalisierung der SF9 Zellen wird ein Kryoröhrchen der SF9-Zellen aus dem Stickstofflagertank in Raum B.OG 2.4.06 entnommen und aufgetaut. Nach dem Ende des Auftauvorgangs werden die Zellen im 15 ml Proberöhrchen durch vorsichtiges Invertieren gleichmäßig verteilt und eine Probe für die Bestimmung der Zellzahl und Vitalität (Lebendzellzahl) entnommen.

Nach Abschluss des Auftauvorganges werden die Zellen in eine Zellkulturflasche geeigneter Größe (T75 oder T175, Suspensionsflaschen 500ml) transferiert, so dass eine Zelldichte von 3 x 10⁵ Zellen pro Milliliter erreicht wird. Die Größe der Zellkulturflasche richtet sich nach der Zahl der eingefrorenen Zellen und deren Vitalität nach dem Auftauen. Im Falle einer T75 wird die Zellsuspension auf ein Volumen >10 und ≤30 ml, im Falle einer T175 Zellkulturflasche wird die Zellsuspension auf ein Volumen von >30 ml und ≤50 ml Vollmedium eingestellt und in die Zellkulturflasche transferiert. Zur Expansion werden die SF9 Zellen in einem Brutschrank bei einer Temperatur von 26 °C und einer Luftfeuchte von ≥ 80 % inkubiert.

### 2. Expansion der Zellen

Die Zellen werden solange im Brutschrank inkubiert, bis der Zellrasen eine Dichte von über 70 % aufweist. Dann werden die Zellen mit PBS Puffer gespült, mit einem sterilen Zellschaber vom Boden des Zellkulturgefäßes abgelöst und in eine größere bzw. mehrere Zellkulturflaschen verteilt. Zum gleichmäßigen Verteilen der Zellen im Vollmedium, wird das Kulturgefäß vorsichtig geschwenkt und anschließend eine zur Bestimmung der Zellzahl entnommen. Die ermittelte Zellzahl legt fest, in welchem Volumen Vollmedium die Zellen in der Folge resuspendiert werden und in welche Größe bzw. in welche Anzahl von Zellkulturflaschen die SF9-Zellen transferiert werden. Hierbei gilt: Es werden jeweils 3x10⁵ Zellen / ml Vollmedium ausgesät. Wobei in eine T75 Flasche 12 bis 30 Milliliter und in eine T175 Flasche 30 bis 50 Milliliter, und 300 bis 500 ml in der Suspensionsflasche Zellsuspension transferiert werden. Einen Tag nach dem Transferieren der SF9 Zellen in neue Zellkulturflaschen wird mit der Vorbereitung der SF9 Zellen zur Infektion mit Baculoviren begonnen.

### 3. Infektion der SF9-Zellen mit Baculoviren

### 3.1. Vorbereitung der SF9-Zellen zur Infektion mit Baculoviren

Vor dem eigentlichen Infektionsprozess werden die SF9-Zellen von ihrer Wachstumsoberfläche abgelöst, die Zahl der vitalen Zellen ermittelt und die Zellen in eine oder mehrere T175 Zellkulturflaschen oder in 500 ml Suspensionsflasche transferiert. Hierzu werden 3 x 10⁷ Zellen in 50 ml Vollmedium pro T175 Zellkulturflasche und 30 bis 50 x 10⁸ pro 500 ml Suspensionsflasche in serumfreies Medium ausgesät.

### 3.2. Infektion der SF9 Zellen mit Baculoviren

16 bis 24 Stunden nach Aussaat der Zellen wird die Zellkulturflasche aus dem Brutschrank genommen, wird das Vollmedium abgenommen und 10 ml vorgewärmtes, serumfreies Medium hinzu gegeben. Jetzt wird die Zellkulturflasche vorsichtig geschwenkt und das serumfreie Medium wieder abgenommen. Damit der Zellrasen nicht austrocknet werden sofort 10 ml serumfreies Medium hinzugegeben und die Menge rekombinanter Baculoviren in Vollmedium pipettiert, so dass eine Multiplizität der Infektion (MOI) von 1 erreicht wird. SF9 Suspensionszellen werden mit den MOI 5 direkt in volles Volumen infiziert. Während der folgenden Infektion werden die SF9-Zellen mit den Baculoviren für 20 ± 5 min bei Raumtemperatur (20 ± 4 °C) inkubiert. Anschließend werden in jede T175 Zellkulturflasche 30 ml serumfreies Zellkulturmedium gegeben und die adhärente oder Suspensionszellen für weitere 5 (± 1) Tage im Brutschrank inkubiert.

### 4. Ernte der VP1-VLP

Zur Ernte der VP1-VLP wird das Zellkulturmedium der infizierten Zellen abgenommen und in ein 50 ml Proberöhrchen transferiert und für 60 min bei 5000xg und 5 °C zentrifugiert, um die enthaltenen Zelltrümmer der lysierten Zellen vom proteinhaltigen Medium zu trennen. Anschließend wird das proteinhaltige Zellkulturmedium (Überstand) in ein geeignetes Becherglas transferiert und bei 5 (± 3) °C gelagert.

### BEISPIEL 3: Aufreinigung der VP1-VLP mittels schwachem Anionenaustauscher

### 1. Aufreinigung der VP1-VLP mittels DEAE-FPLC

Die VP1-VLP im Zellkulturmedium werden mithilfe der Crossflow Filtration (Vivoflow System Easy Load von Sartorius) aufgereinigt und im gleichen Prozeßschritt auch aufkonzentriert. Das Crossflow System wird für die Benutzung vorbereitet. 500mL ddH₂0 werden in ein passendes Reservoir platziert und das System damit gespült. Der Druckbereich liegt bei 1,5 bis 2,5 bar. 500mL 10mM Tris-HCI wird in dem Reservoir platziert, das System wird damit gespült. Der Druckbereich liegt bei 1,5 bis 2,5 bar.

Der Zellkulturüberstand wird mit einem Zentrifugationsschritt von möglichen Zelltrümmern befreit. Der geklärte Zellkulturüberstand wird in das Reservoir überführt und durch das System gepumpt. Das Ursprungsvolumen wird bis zur Hälfte reduziert und dann ein größeres Reservoir (in der Regel mit einem Volumen von 1 bis 2L) mit dem Standardpuffersystem (10 mM Tris-HCI, 100 mM NaCl) angeschlossen. Anschließend wird das Medium mit Tris-HCI Puffersystem (10 mM Tris-HCI, 100 mM NaCl) ausgetauscht. Das Volum wird bis 150mL reduziert. Durch diese Crossflow Filtration werden alle niedermolekularen Verunreinigungen bis hin zu Proteinen und Proteinfragmenten mit einer Größe von 100kDa entfernt.

In der Probe liegen die VLP zunächst als intakte Partikel vor. Durch die folgende Anionenaustauscher-Chromatographie werden die in der Probe enthaltenden Proteinkontaminationen und freie Nukleinsäuren abgetrennt. Hierbei wird eine DEAE-Sepharose Säule als schwacher Ionenaustauscher verwendet. Die VLP werden im Standardpuffer mithilfe eines lineare NaCI-Gradienten (100 mM bis 1M NaCl) oder durch Stufengradienten (3 Schritte: 1. 50 bis 150mM NaCl, 2. 200 bis 400 mM NaCl und 3. 1M bis 2M NaCl; 4 Schritte: 1. 50 bis 150mM NaCl, 2. 200 bis 400 mM NaCl, 3. 500mM bis 800mM NaCl und 4. 1M bis 2M NaCl) eluiert.

### 2. Optionale Nachreinigung mittels Dialyse

Optional wird die VLP-haltige Probe dann 24 ± 2 Stunden gegen 10 mM Tris-HCI, 50 bis 150 mM NaCl, pH 7.5 dialysiert um die Salzkonzentration korrekt einzustellen.

### 3. Lagerung der VP1-VLP

Die VP1-VLP werden mit 10mM Tris-HCI, 150mM NaCl, pH 7,5 auf die Konzentration von 0,5µg/µl verdünnt, in 1,5ml Proberöhrchen aliquotiert und bei -80± 5°C gelagert.

### BEISPIEL 4: Aufreinigung der VP1-VLP mittels starkem Anionenaustauscher

### 1. Aufreinigung der VP1-VLP mittels Q-Sepharose-FPLC

Die VP1-VLP im Zellkulturmedium werden mithilfe der Crossflow Filtration (Vivoflow System Easy Load von Sartorius) aufgereinigt und im gleichen Prozeßschritt auch aufkonzentriert. Das Crossflow System wird für die Benutzung vorbereitet. 500mL ddH₂0 werden in ein passendes Reservoir platziert und das System damit gespült. Der Druckbereich liegt bei 1,5 bis 2,5 bar. 500mL 10mM Tris-HCI wird in dem Reservoir platziert, das System wird damit gespült. Der Druckbereich liegt bei 1,5 bis 2,5 bar.

Der Zellkulturüberstand wird mit einem Zentrifugationsschritt von möglichen Zelltrümmern befreit. Der geklärte Zellkulturüberstand wird in das Reservoir überführt und durch das System gepumpt. Das Ursprungsvolumen wird bis zur Hälfte reduziert und dann ein größeres Reservoir (in der Regel mit einem Volumen von 1 bis 2L) mit dem Standardpuffersystem (10 mM Tris-HCI, 100 mM NaCl) angeschlossen. Anschließend wird das Medium mit Tris-HCI Puffersystem (10 mM Tris-HCI, 100 mM NaCl) ausgetauscht. Das Volum wird bis 150mL reduziert. Durch diese Crossflow Filtration werden alle niedermolekularen Verunreinigungen bis hin zu Proteinen und Proteinfragmenten mit einer Größe von 100kDa entfernt.

In der Probe liegen die VLP zunächst als intakte Partikel vor. Durch die folgende Anionenaustauscher-Chromatographie werden die in der Probe enthaltenden Proteinkontaminationen und freie Nukleinsäuren abgetrennt. Hierbei wird eine Q-Sepharose Säule als starker Ionenaustauscher verwendet. Die VLP werden im Standardpuffer mithilfe eines lineare NaCI-Gradienten (100 mM bis 1M NaCl) oder durch Stufengradienten (3 Schritte: 1. 50 bis 150mM NaCl, 2. 200 bis 400 mM NaCl und 3. 1M bis 2M NaCl; 4 Schritte: 1. 50 bis 150mM NaCl, 2. 200 bis 400 mM NaCl, 3. 500mM bis 800mM NaCl und 4. 1M bis 2M NaCl) eluiert.

### 2. Optionale Nachreinigung mittels Dialyse

Optional wird die VLP-haltige Probe dann 24 ± 2 Stunden gegen 10 mM Tris-HCI, 50 bis 150 mM NaCl, pH 7.5 dialysiert um die Salzkonzentration korrekt einzustellen.

### 3. Lagerung der VP1-VLP

Die VP1-VLP werden mit 10mM Tris-HCI, 150mM NaCl, pH 7,5 auf die Konzentration von 0,5µg/µl verdünnt, in 1,5ml Proberöhrchen aliquotiert und bei -80± 5°C gelagert.

### BEISPIEL 5: Aufreinigung der VP1-Pentameren mittels schwachem Anionenaustauscher

### 1. Dissoziation der VLP in VP1-Pentamere

Im ersten Schritt werden die VLP in der Probe durch Zugabe von 5 bis 20 mM DTT und 10-30 mM EGTA (Endkonzentration) bei Raumtemperatur für 1 Stunde dissoziiert und auf eine Säule mit einer DEAE-Matrix als schwachen Anionenaustauscher mit DEAE-Matrix aufgetragen. Dieser Schritt ist essentiell um eine nahezu 100%ige Reinheit der VP1-Pentameren zu erhalten, da in diesen Schritt werden die verpackte Nukleinsäuren und andere Kontaminationen eliminiert. Die Pentameren werden mit einem lineare NaCl-Gradienten (100 mM bis 1M NaCl), oder durch Stufengradienten (3 Schritte: 1. 50 bis 150mM NaCl, 2. 200 bis 400 mM NaCl und 3. 1M bis 2M NaCl; 4 Schritte: 1. 50 bis 150mM NaCl, 2. 200 bis 400 mM NaCl, 3. 500mM bis 800mM NaCl und 4. 1M bis 2M NaCl) eluiert.

### 2. Optionale Nachreinigung mittels Dialyse

Für den Fall, dass eine Reduzierung der Salzkonzentration erforderlich ist kann die VP1-Pentamere enthaltende Lösung optional über Nacht bei 5 ± 3 °C gegen physiologische Kochsalzlösung dialysiert werden. Bei einer Verpackung von Wirkstoffen werden die VP1-Pentamere ohne diesen optionalen Nachreinigungsschritt mit dem Wirkstoff vermischt (s. folgenden Schritt 3).

### 3. Mischen der VP1-Pentamere mit den Wirkstoffen

1µg bis 100µg aufgereinigten VP1-Pentameren werden mit 1 bis 10µg Plasmide DNA vermischt. Die Mischung wird 15 min bei Raumtemperatur inkubiert. Die Mischung wird in Dialysekammer (zum Beispiel von Pierce) mit Porengrößen zwischen 5kDa und 20kDa platziert. Die Dialysekammern werden in Reassoziationspuffer platziert.

### 4. Reassoziation der VP1-Pentamere zu den VLP

Die Reassoziation der VP1-Pentamere zu den VLP erfolgt durch Dialyse gegen einen sogenannten Reassoziationspuffer, enthaltend 1 bis 5 mM CaCl₂, 10 mM Tris-HCI und 150 mM NaCl, pH 7,5) bei 5 ± 3°C über 24-48 h.

### 5. Lagerung der Wirkstoff-haltigen VLP

Danach werden die VLP bei 5 ± 3°C bis zur Weiterverarbeitung gelagert oder zur dauerhaften Lagerung bei -80°C eingefroren.

### BEISPIEL 6: Aufreinigung der VP1-Pentamere mittels starkem Anionenaustauscher

Die VP1-VLP im Zellkulturmedium werden mithilfe der Crossflow Filtration (Vivoflow System Easy Load von Sartorius) aufgereinigt und im gleichen Prozeßschritt auch aufkonzentriert. Das Crossflow System wird für die Benutzung vorbereitet. 500mL ddH₂0 werden in ein passendes Reservoir platziert und das System damit gespült. Der Druckbereich liegt bei 1,5 bis 2,5 bar. 500mL 10mM Tris-HCI wird in dem Reservoir platziert, die System wird damit gespült. Der Druckbereich liegt bei 1,5 bis 2,5 bar.

Der Zellkulturüberstand wird mit einem Zentrifugationsschritt von Zelltrümmern befreit. Der geklärte Zellkulturüberstand wird in das Reservoir überführt und durch das System gepumpt. Das Ursprungsvolumen wird bis zur Hälfte reduziert und dann ein größeres Reservoir (in der Regel mit einem Volumen von 1 bis 2L) mit dem Standardpuffersystem (im Regel wird als Standardpuffer 10 mM Tris-HCI, 100 mM NaCl benutzt) angeschlossen. Anschließend wird das Medium mit Tris-HCI Puffersystem (im Regel wird als Standardpuffer 10 mM Tris-HCI, 100 mM NaCl benutzt) ausgetauscht. Das Volumen wird bis 150mL reduziert. Durch diese Crossflow Filtration werden alle niedermolekularen Verunreinigungen bis hin zu Proteinen und Proteinfragmenten mit einer Größe von 100kDa entfernt.

### 2. Dissoziation der VLP in VP1-Pentamere

Im ersten Schritt werden die VLP in der Probe durch Zugabe von 5 bis 20 mM DTT und 10-30 mM EGTA (Endkonzentration) bei Raumtemperatur für 1 Stunde dissoziiert und auf eine Mono Q-Säule oder Q-Sepharose-Säule aufgetragen. Dieser Schritt ist essentiell um eine nahezu 100%ige Reinheit der VP1-Pentameren zu erhalten, da in diesen Schritt werden die verpackte Nukleinsäuren und andere Kontaminationen eliminiert. Die Pentameren werden mit einem lineare NaCI-Gradienten (100 mM bis 1M NaCl), oder durch Stufengradienten (3 Schritte: 1. 50 bis 150mM NaCl, 2. 200 bis 400 mM NaCl und 3. 1M bis 2M NaCl; 4 Schritte: 1. 50 bis 150mM NaCl, 2. 200 bis 400 mM NaCl, 3. 500mM bis 800mM NaCl und 4. 1M bis 2M NaCl) eluiert.

### 3. Optionale Nachreinigung mittels Dialyse

Für den Fall, dass eine Reduzierung der Salzkonzentration erforderlich ist kann die VP1-Pentamere enthaltende Lösung optional über Nacht bei 5 ± 3 °C gegen physiologische Kochsalzlösung dialysiert werden. Bei einer Verpackung von Wirkstoffen werden die VP1-Petnamere ohne diesen optionalen Nachreinigungsschritt mit dem Wirkstoff vermischt (s. folgenden Schritt 3).

### 4. Mischen der VP1-Pentamere mit den Wirkstoffen

1µg bis 100µg der aufgereinigten VP1-Pentamere werden mit 1 bis 10µg Plasmid DNA vermischt. Die Mischung wird 15 min bei Raumtemperatur inkubiert. Die Mischung wird in einer Dialysekammer (zum Beispiel von Pierce) mit Porengrößen zwischen 5kDa und 20kDa platziert. Die Dialyse erfolgt gegen einen Reassoziationspuffer.

### 5. Reassoziation der VP1-Pentamere zu den VLP

Die Reassoziation der VP1-Pentamere zu den VLP erfolgt durch Dialyse gegen einen sogenannten Reassoziationspuffer, enthaltend 1 bis 5 mM CaCl₂, 10 mM Tris-HCI und 150 mM NaCl, pH 7,5) bei 5 ± 3°C über 24-48 h.

### 6. Lagerung der Wirkstoff-haltigen VLP

Danach werden die VLP bei 5 ± 3°C bis zur Weiterverarbeitung gelagert oder zur dauerhaften Lagerung bei -80°C eingefroren oder alternativ lyophilisiert.

### Abkürzungen

- Bacmid: = Shuttle Vektor, in E.coli und in Insektenzellen propagierbar
- FCS: = Fötales Kälberserum
- LAF: = Laminar Air Flow
- min: = Minuten
- rpm: = Umdrehungen pro Minute
- RT: = Raumtemperatur
- T25, T75, T175: = Zellkulturflasche mit 25, 75 bzw. 175 cm² Wachstumsfläche
- Vollmedium: = TC-100 Insekt Medium mit 10 % foetalem Kälberserum
- VLP: = Virus like partikel
- VP1: = Virus Protein 1 des JC Virus

### Verzeichnis der Abbildungen

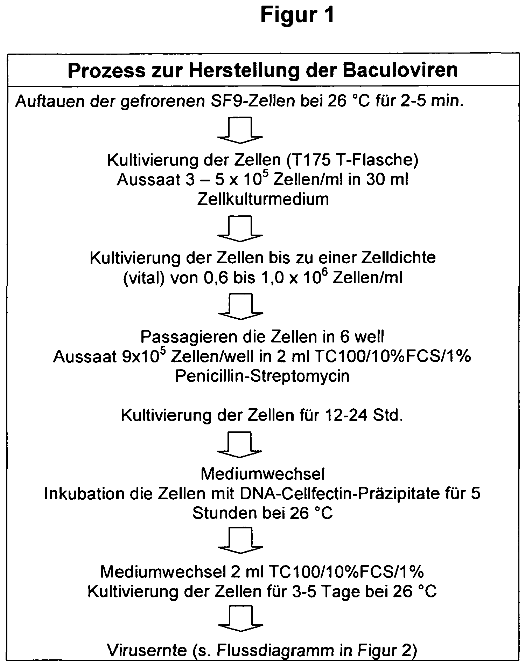

- **Fig. 1:**: Flußdiagramm zur Herstellung VP1-kodierender Baculoviren durch Transfektion von SF9-Insektenzellen

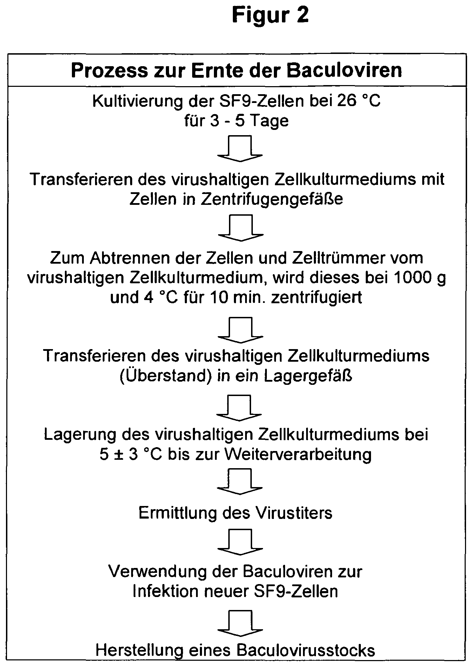

- **Fig. 2:**: Flußdiagramm zur Ernte der Baculoviren zur Erstellung eines "seed strain"

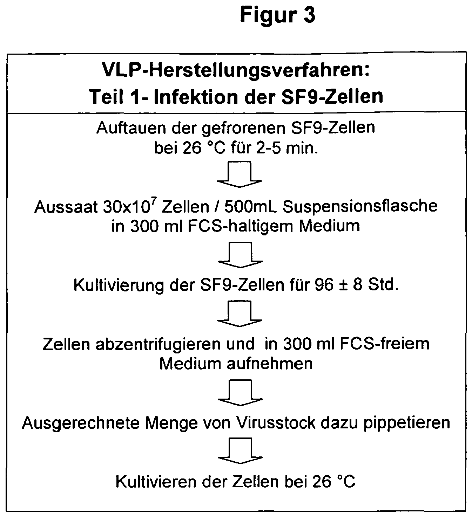

- **Fig. 3:**: Flußdiagramm zum VLP-Herstellungsverfahren: Teil 1- Infektion der SF9-Zellen

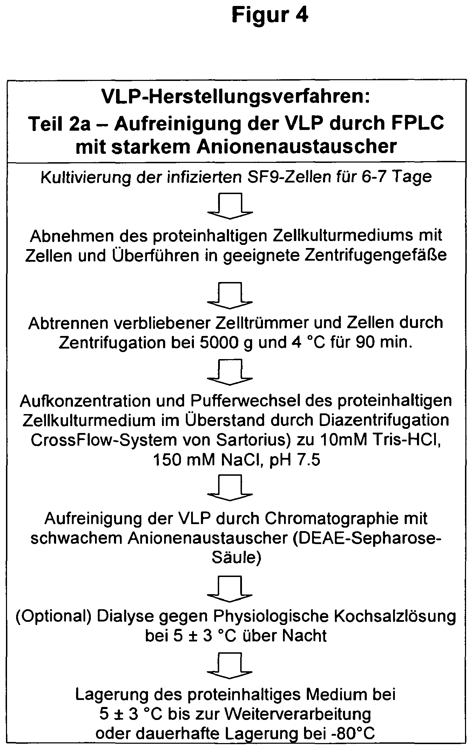

- **Fig. 4:**: Flußdiagramm zum VLP-Herstellungsverfahren: Teil Ila Aufreinigung der VLP durch FPLC mit starkem Anionenaustauscher

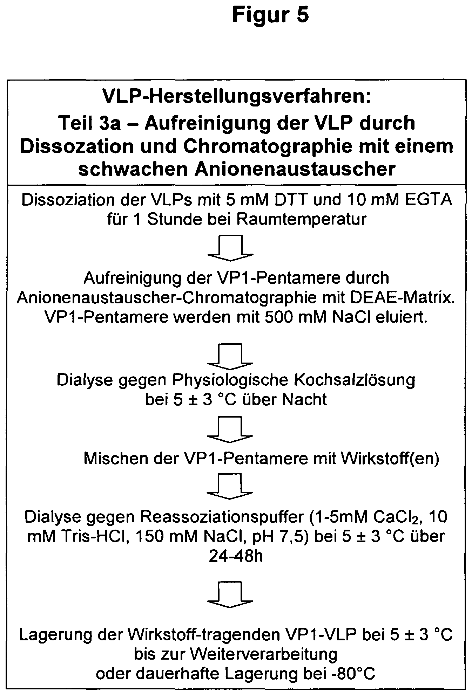

- **Fig. 5:**: Flußdiagramm zum VLP-Herstellungsverfahren: Teil 3a Dissoziation der der VLP und Aufreinigung der Pentamere mittels schwachem Anionenaustauscher

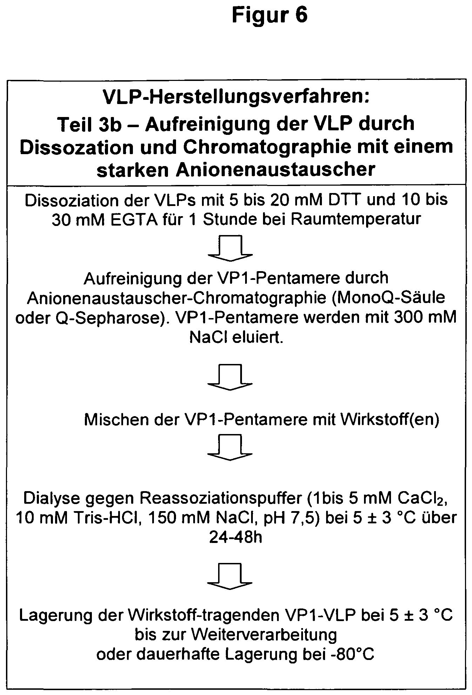

- **Fig. 6:**: Flußdiagramm zum VLP-Herstellungsverfahren: Teil 3b Aufreinigung der VLP durch Dissocation und Chromatographie mit einem starkenAnionenaustauscher

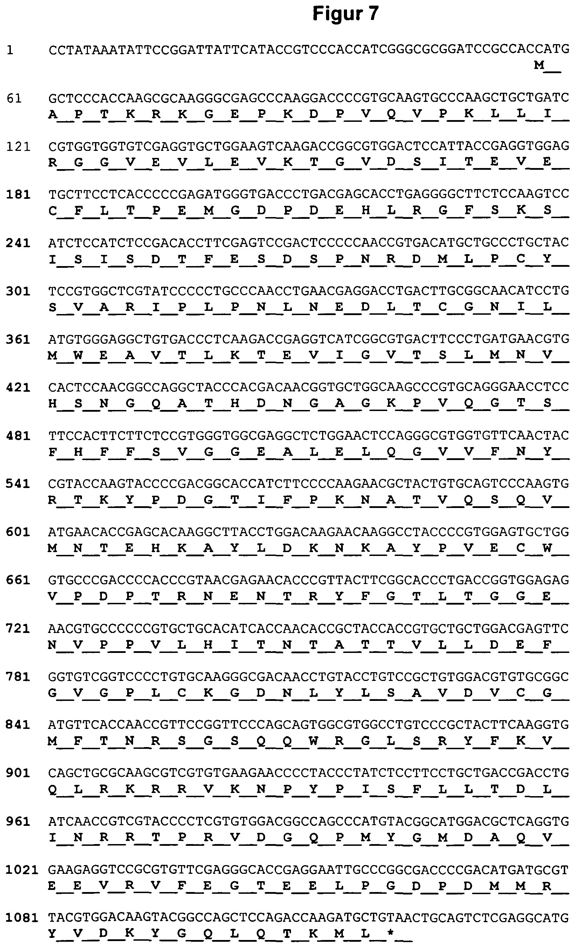

- **Fig. 7:**: Aminosäuresequenz des VP1 Kapsidproteins abgeleitet vom Polyoma Virus JC nebst der hierfür kodierenden Nukleotidsequenz, die für die Expression in Insektenzellen kodonoptimiert wurde.

## Patentansprüche

1. Verfahren zur Aufreinigung von Virus-ähnlichen Partikeln (VLP), **dadurch gekennzeichnet, dass** eine VLP-enthaltende Zusammensetzung über ein Filtermedium mit einer Ausschlussgrenze von mehr als 30 kDa bis 1500 kDa filtriert wird, wobei als VLP-enthaltende Zusammensetzung der Überstand der Kultur der VLP exprimierenden Zellen (Zellkulturüberstand) verwendet wird, wobei die VLP aus dem Strukturprotein VP1 des humanen JC-Virus aufgebaut sind und mit der Filtration gleichzeitig eine Umpufferung der VLP-enthaltenden Zusammensetzung erfolgt.

2. Verfahren gemäß Anspruch 1, wobei das Filtermedium eine Ausschlussgrenze von mehr als 30 kDa bis 100 kDa aufweist.

3. Verfahren gemäß Anspruch 1 oder 2, wobei beim Filtrieren eine Druckdifferenz zwischen 0,5 bar und 10 bar, bevorzugt zwischen 0,5 und 5 bar und besonders bevorzugt zwischen 0,5 bar und 3 bar aufgebaut wird.

4. Verfahren gemäß einem der vorherigen Ansprüche, wobei bei der Umpufferung der pH-Wert auf zwischen 5,0 und 8,0 abgesenkt wird.

5. Verfahren gemäß einem der vorherigen Ansprüche, wobei die Filtration als Crossflow-Filtration durchgeführt wird.

6. Verfahren gemäß Anspruch 5, wobei bei der Crossflow-Filtration eine Querströmung von 2,5 bis 3 m/s erzeugt wird, die entlang des Filtermediums fließt.

7. Verfahren gemäß einem der vorherigen Ansprüche, wobei mit der Filtration gleichzeitig eine Aufkonzentrierung der VLP-enthaltenden Zusammensetzung erfolgt.

8. Verfahren gemäß einem der vorherigen Ansprüche, wobei der Zellkulturüberstand vor der Filtration zentrifugiert, aber nicht enzymatisch oder chemisch behandelt oder chromatographiert wird.

9. Verfahren gemäß einem der vorherigen Ansprüche, wobei die VLP zusätzlich den folgenden Schritten unterzogen werden:
(a) Dissoziation der VLP;
(b) Aufreinigung der dissoziierten VLP;
(c) Reassoziation der VLP.

10. Verfahren gemäß einem der vorherigen Ansprüche, **dadurch gekennzeichnet, dass** die VLP enthaltende Zusammensetzung nach der Filtration zusätzlich chromatographisch, vorzugsweise über eine Anionenaustausch-Chromatographie aufgereinigt wird.

11. Verfahren nach einem der vorherigen Ansprüche, **dadurch gekennzeichnet, dass** das VLP das Strukturprotein VP1 mit einer Aminosäuresequenz codiert von einer Nukleotidsequenz gemäß SEQ. ID. NO:1 oder SEQ. ID. NO:2 aufweist.

## Claims

1. A method for the purification of virus-like particles (VLP), **characterised in that** a VLP-containing composition is filtered through a filter medium with an exclusion limit of more than 30 kDa to 1500 kDa, wherein the supernatant of the culture of VLP-expressing cells (cell culture supernatant) is used as VLP-containing composition, wherein the VLP are composed of the structural protein VP1 of the human JC virus and a buffer exchange of the VLP-containing composition is done at the same time as the filtration.

2. The method according to claim 1, wherein the filter medium has an exclusion limit of more than 30 kDa to 100 kDa.

3. The method according to claim 1 or 2, wherein a pressure difference of between 0.5 bar and 10 bar, preferably of between 0.5 and 5 bar and particularly preferably of between 0.5 bar and 3 bar, is built up during filtration.

4. The method according to any of the preceding claims, wherein the pH value is reduced to between 5.0 and 8.0 during the buffer exchange.

5. The method according to any of the preceding claims, wherein the filtration is carried out as cross flow filtration.

6. The method according to claim 5, wherein a cross flow of 2.5 to 3 m/s which flows along the filter medium is generated during cross flow filtration.

7. The method according to any of the preceding claims, wherein the VLP-containing composition is concentrated at the same time as filtration.

8. The method according to any of the preceding claims, wherein before filtration, the cell culture supernatant is centrifuged, but not enzymatically or chemically treated or chromatographed.

9. The method according to any of the preceding claims, wherein the VLP are additionally subjected to the following steps:
(a) Dissociation of the VLP;
(b) Purification of the dissociated VLP;
(c) Reassociation of the VLP.

10. The method according to any of the preceding claims, **characterised in that** after filtration, the VLP-containing composition is additionally purified using chromatography, preferably using anion exchange chromatography.

11. The method according to any of the preceding claims, **characterised in that** the VLP has the structural protein VP1 with an amino acid sequence coded by a nucleotide sequence according to SEQ. ID. NO:1 or SEQ. ID. NO:2.

## Revendications

1. Procédé de purification de particules analogues à des virus (VLP), **caractérisé en ce qu'**une composition contenant des VLP est filtrée sur un milieu de filtration ayant une limite d'exclusion de plus de 30 kDa à 1 500 kDa, le surnageant de la culture des cellules exprimant les VLP (surnageant de culture cellulaire) étant utilisé en tant que composition contenant des VLP, les VLP étant formées par la protéine structurale VP1 du virus JC humain, et un tamponnage de la composition contenant des VLP ayant lieu simultanément avec la filtration.

2. Procédé selon la revendication 1, dans lequel le milieu de filtration présente une limite d'exclusion de plus de 30 kDa à 100 kDa.

3. Procédé selon la revendication 1 ou 2, dans lequel une différence de pression comprise entre 0,5 bar et 10 bar, de préférence entre 0,5 et 5 bar, et de manière particulièrement préférée entre 0,5 bar et 3 bar, est établie lors de la filtration.

4. Procédé selon l'une quelconque des revendications précédentes, dans lequel le pH est réduit à une valeur comprise entre 5,0 et 8,0 lors du tamponnage.

5. Procédé selon l'une quelconque des revendications précédentes, dans lequel la filtration est réalisée sous la forme d'une filtration à courant transversal.

6. Procédé selon la revendication 5, dans lequel un écoulement transversal de 2,5 à 3 m/s, qui s'écoule le long du milieu de filtration, est généré lors de la filtration à courant transversal.

7. Procédé selon l'une quelconque des revendications précédentes, dans lequel une concentration de la composition contenant des VLP a lieu simultanément avec la filtration.

8. Procédé selon l'une quelconque des revendications précédentes, dans lequel le surnageant de culture cellulaire est centrifugé avant la filtration, mais n'est pas traité enzymatiquement ou chimiquement ou chromatographié.

9. Procédé selon l'une quelconque des revendications précédentes, dans lequel les VLP sont en outre soumises aux étapes suivantes :
(a) la dissociation des VLP ;
(b) la purification des VLP dissociées ;
(c) la réassociation des VLP.

10. Procédé selon l'une quelconque des revendications précédentes, **caractérisé en ce que** la composition contenant des VLP est en outre purifiée chromatographiquement, de préférence par une chromatographie à échange d'anions, après la filtration.

11. Procédé selon l'une quelconque des revendications précédentes, **caractérisé en ce que** la VLP comprend la protéine structurale VP1 ayant une séquence d'acides aminés codée par une séquence nucléotidique selon SEQ ID NO : 1 ou SEQ ID NO : 2.
